# EUROPEAN PATENT SPECIFICATION

(11) **EP 2 334 804 B1**
(45) Date of publication and mention of the grant of the patent: **04.12.2013**
(21) Application number: 09819852.6
(22) Date of filing: 07.10.2009
(51) Int. Cl.: C12Q 1/02, C12N 5/071, G01N 33/15, G01N 33/50, C12N 5/0793

(54) **CO-CULTURE COMPOSITIONS AND METHODS**
KOKULTURZUSAMMENSETZUNGEN UND -VERFAHREN
COMPOSITIONS ET PROCÉDÉS POUR CO-CULTURE

(30) Priority: 07.10.2008 US 103509 P
(43) Date of publication of application: 22.06.2011
(73) Proprietor: True North Therapeutics, Inc., South San Francisco, CA 94080 (US)
(72) Inventor: ELIAS, Guillermo, Muñoz, South San Francisco CA 94080 (US); JAVAHERIAN, Ashkan, South San Francisco CA 94080 (US); IRION, Stefan, South San Francisco CA 94080 (US)
(74) Representative: Wilkinson, Marc George
(86) International application number: PCT/US2009/059910
(87) International publication number: WO 2010/042669

(56) References cited:
- WO-A2-2009/007852
- STORMY J CHAMBERLAIN ET AL: "Induced pluripotent stem (iPS) cells as in vitro models of human neurogenetic disorders", NEUROGENETICS, SPRINGER, BERLIN, DE, vol. 9, no. 4, 13 September 2008 (2008-09-13), pages 227-235, XP019635665, ISSN: 1364-6753, DOI: 10.1007/S10048-008-0147-Z
- MAKIKO NAGAI ET AL: "Astrocytes expressing ALS-linked mutated SOD1 release factors selectively toxic to motor neurons", NATURE NEUROSCIENCE, vol. 10, no. 5, 15 April 2007 (2007-04-15) , pages 615-622, XP55024284, ISSN: 1097-6256, DOI: 10.1038/nn1876
- FRANCESCO PAOLO DI GIORGIO ET AL: "Non-cell autonomous effect of glia on motor neurons in an embryonic stem cell-based ALS model", NATURE NEUROSCIENCE, vol. 10, no. 5, 15 April 2007 (2007-04-15) , pages 608-614, XP55024285, ISSN: 1097-6256, DOI: 10.1038/nn1885
- PARK IN-HYUN ET AL: "Disease-specific induced pluripotent stem cells", CELL, CELL PRESS, US, vol. 134, no. 5, 5 September 2008 (2008-09-05), pages 877-886, XP002571839, ISSN: 0092-8674, DOI: 10.1016/J.CELL.2008.07.041
- DIMOS J T ET AL: "Induced pluripotent stem cells generated from patients with ALS can be differentiated into motor neurons", SCIENCE, AMERICAN ASSOCIATION FOR THE ADVANCEMENT OF SCIENCE, WASHINGTON, DC; US, vol. 321, no. 5893, 29 August 2008 (2008-08-29), pages 1218-1221, XP002571838, ISSN: 0036-8075, DOI: 10.1126/SCIENCE.1158799 [retrieved on 2008-07-31]
- LOWRY W E ET AL: "Generation of human induced pluripotent stem cells from dermal fibroblasts", PROCEEDINGS OF THE NATIONAL ACADEMY OF SCIENCES, NATIONAL ACADEMY OF SCIENCES, vol. 105, no. 8, 26 February 2008 (2008-02-26), pages 2883-2888, XP002555951, ISSN: 0027-8424, DOI: 10.1073/PNAS.0711983105 [retrieved on 2008-02-15]
- I.-H. PARK ET AL.: 'Disease-specific induced pluripotent stem(iPS) cells.' CELL. vol. 134, no. 5, 05 September 2008, pages 877 - 886, XP002571839
- J. T. DIMOS ET AL.: 'Induced pluripotent stem cells generated from patients with ALS can be differentiated into motor neurons.' SCIENCE. vol. 321, 29 August 2008, pages 1218 - 1221, XP002571838
- M. WERNIG ET AL.: 'Neurons derived from reprogrammed fibroblasts functionally integrate into the fetal brain and improve symptoms of rats with Parkinson's disease.' PNAS. vol. 105, no. 15, 15 April 2008, pages 5856 - 5861, XP008145440

## Description

### BACKGROUND OF THE INVENTION

The lack of robust in vitro disease models has been a significant impediment to understanding disease mechanisms and to the development of effective therapeutic agents. In particular, there has been a lack of *in vitro* models that include disease-relevant, differentiated cells derived from actual patients, for example dopaminergic neurons from Parkinson's patients, pancreatic P cells from diabetics, or hippocampal neurons from sporadic Alzheimer's patients, cultured in a way that recapitulates disease-relevant cellular phenotypes amenable to drug screening. The lack of suitable in vitro models has been especially problematic for sporadic human diseases where relevant mutations/genotypes are unknown. However, even where single specific disease-causing mutations are identified and "engineered' into a human embryonic stem cell line, the resulting models are unlikely to capture cellular phenotypes relevant to disease as manifested in patients. This is due to the fact that disease manifestation, even in patients suffering from a monogenic disorder, will not merely depend on the underlying single-gene mutation, but on the interaction of that mutation with the varied genetic backgrounds of the patients. Indeed, in many cases patients with identical disease gene mutations have widely varying clinical manifestations of the disease, and, very importantly, may respond very differently to the same therapeutic agent.

Nagai et al., Nature Neuroscience, vol 10 no 5 p 615-62, describes culturing mouse primary spinal neurons (PMN) or mouse embryonic stem cell-derived motor neurons (ESMN) on a mouse astrocyte monolayer (AML), where the PMN or ESMN or the astrocytes were genetically modified to express a mutated form of human superoxide dismutase-1 (SOD1). Paolo di Giorgio et al., Nature Neuroscience, vol 10 no 5 p 608-614 describes culturing motor neurons, generated from mouse embryonic stem cells (ESCs) that had a wild-type or mutant allele of the human SOD 1 gene, with mouse primary glial cells which had the mutant allele of the SOD1 gene.

### SUMMARY OF THE INVENTION

This disclosure provides compositions of cellular co-cultures that comprise differentiated cells derived from induced pluripotent stem cells (iPS cells) and methods of using such co-cultures for identifying therapeutic compounds.

The present invention provides a method according to claim 1 and a co-culture composition according to claim 12.

In a first aspect, the present invention provides a method of identifying a modulatory agent, the method comprising: (a) co-culturing a first and a second population of differentiated cells in the presence or absence of a test agent, wherein the first population comprises differentiated cells derived from induced pluripotent stem cells (iPSCs) of a first human subject and wherein the second population comprises differentiated cells derived from a second human subject, wherein the differentiated cells in at least one of said populations comprise neurons, neural stem cells, or neural progenitors; and (b) determining that the test agent is a modulatory agent if a cellular phenotype of the co-cultured differentiated cells of the first or second population is reduced or increased in the presence of the test agent, wherein the first or second human subject is identified as having, or predisposed to a neurodegenerative disorder. In another embodiment of this aspect, the second population comprises differentiated cells derived from induced pluripotent stem cells from the second human subject. In yet another embodiment of this aspect, the second population does not comprise cells differentiated from human embryonic stem cells. In some embodiments of this aspect, the neurodegenerative disorder does not comprise ALS caused by a SOD^{G37R} mutation. In some embodiments of this aspect, the neurodegenerative disorder does not comprise ALS caused by a SOD1^{G93A}, SOD1^{G85R}, or SOD1^{G37R} mutation. In some embodiments of this aspect, in the co-cultured first and second populations the ratio of the differentiated cells of one cell type from the first population to differentiated cells of the one cell type from the second population is greater than 1:1, 10:1, 100:1, 1000:1, or 10,000:1. In some embodiments of this aspect, in the co-cultured first and second populations the ratio of the differentiated cells of one cell type from the first population to differentiated cells of the one cell type from the second population is less than 1:1, 10:1, 100:1, 1000:1, or 10,000:1.

In this first aspect, the first subject is identified as having, or predisposed to a neurodegenerative disorder. In some embodiments of this aspect, the second subject is identified as having, or predisposed to a neurodegenerative disorder. In some embodiments of this aspect, the neurodegenerative disorder is a sporadic form of a neurodegenerative disorder. In some embodiments of this aspect, at least one of the populations is detectably labeled. In some embodiments of this aspect, both of the populations are detectably labeled. In some embodiments of this aspect, at least one population comprises cells detectably labeled by expression of a fluorescent protein, staining with a fluorescent dye, staining with a fluorescently labeled antibody or staining with a fluorescently labeled protein with high affinity for a cell surface protein or receptor. In some embodiments of this aspect, the method further comprises co-culturing a third population of cells comprising differentiated cells of a cell type enriched relative to the first and second populations. In some embodiments of this aspect, the differentiated cells comprise myocytes fused to form myotubes. In some embodiments of this aspect, the cellular phenotype is a synaptic phenotype, an axonal morphology phenotype, or a dendritic morphology phenotype. In some embodiments of this aspect, the first and second population is substantially enriched in a cell type relative to the other population. In some embodiments of this aspect, the enriched cell-type is an astrocyte, an oligodendrocyte, a motor neuron, a dopaminergic neuron, a cholinergic neuron, a glutamatergic neuron, a serotonergic neuron, a GABAergic interneuron, a myocyte, or a cardiomyocyte. In some embodiments of this aspect, the cellular phenotype is one or more phenotypes selected from the group consisting of: survival, apoptosis, necrosis, axonal degeneration, axonal guidance, axonal morphology, dendritic morphology, receptor density, synaptogenesis, neurogenesis, synapse density, synaptic transmission, synaptic signaling, receptor trafficking, protein trafficking, protein aggregation, proteasome activity, receptor expression, oxidative stress (ROS), FGFR-signaling, FGF signaling, mitochondrial activity, mitochondrial distribution, mitochondrial morphology, and mRNA expression profile.

In some embodiments of this first aspect, differentiated cells in the first and second populations of cells comprise neurons. In some embodiments of this aspect, the differentiated cells in the first and second populations of cells comprise motor neurons. In some embodiments of this aspect, the neurodegenerative disorder is Amyotrophic Lateral Sclerosis (ALS) or Spinal Muscular Atrophy (SMA). In some embodiments of this aspect, the neurodegenerative disorder is ALS and the ALS is a sporadic form of ALS. In some embodiments of this aspect, the method further comprises culturing the differentiated cells in the presence of muscle tissue derived from a healthy subject. In some embodiments of this aspect: (a) the differentiated cells in the first population of cells comprise GABAergic neurons and the differentiated cells in the second population of cells comprise dopaminergic neurons or (b) the differentiated cells in the first population of cells comprise dopaminergic neurons and the differentiated cells in the second population of cells comprise GABAergic neurons. In some embodiments of this aspect, the neurodegenerative disorder is Parkinson's Disease or a sporadic or familial form of Parkinson's Disease. In some embodiments of this aspect, either: (a) the differentiated cells in the first population of cells comprise motor neurons and the differentiated cells in the second population of cells comprise astrocytes or (b) the differentiated cells in the first population of cells comprise astrocytes and the differentiated cells in the second population of cells comprise motor neurons. In some embodiments of this aspect, the neurodegenerative disorder is Huntington's Disease or Amyotrophic Lateral Sclerosis (ALS).

In some embodiments of this aspect either: (a) the differentiated cells in the first population of cells comprise cortical projection neurons (CPNs) and the differentiated cells in the second population of cells comprise spinal cord motor neurons (SCMNs) or (b) the differentiated cells in the first population of cells comprise SCMNs and the differentiated cells in the second population of cells comprise CPNs.

In some embodiments of this aspect, the differentiated cells in the first population comprise neurons selected from the group consisting of: motor neurons, dopaminergic neurons, GABAergic neurons, cortical projection neurons, striatal neurons, and spinal cord motor neurons. In some embodiments of this aspect, the differentiated cells in the second population comprise neurons selected from the group consisting of: motor neurons, dopaminergic neurons, GABAergic neurons, cortical projection neurons, striatal neurons, and spinal cord motor neurons. In some embodiments of this aspect, the neurodegenerative disorder is selected from the group consisting of: Amyotrophic Lateral Sclerosis (ALS), Spinal Muscular Atrophy (SMA), Huntington's Disease, and Parkinson's Disease. In some embodiments of this aspect, the neurodegenerative disorder is selected from the group consisting of: Alexander's disease, Alper's disease, Alzheimer's disease, amyotrophic lateral sclerosis, ataxia telangiectasia, Batten disease, bovine spongiform encephalopathy, Canavan disease, Cockayne syndrome, corticobasal degeneration, Creutzfeldt-Jakob disease, Huntington's disease, HIV-associated dementia, Kennedy's disease, Krabbe's disease, lewy body dementia, Machado-Joseph disease, multiple sclerosis, multiple system atrophy, narcolepsy, neuroborreliosis, Parkinson's disease, Pelizaeus-Merzbacher Disease, Pick's disease, primary lateral sclerosis, prion diseases, Refsum's disease, Sandhoffs disease, Schilder's disease, subacute combined degeneration of spinal cord secondary to pernicious anaemia, schizophrenia, spinocerebellar ataxia, spinal muscular atrophy, Steele-Richardson-Olszewski disease, and tabes dorsalis.

In another aspect, this disclosure provides method of identifying a modulatory agent, the method comprising: (a) co-culturing a first and a second population of differentiated cells in the presence or absence of a test agent, wherein the first population comprises differentiated cells derived from induced pluripotent stem cells (iPSCs) of a first human subject and wherein the second population comprises differentiated cells derived from a second human subject, wherein the differentiated cells in at least one of said populations comprise neurons, neural stem cells, or neural progenitors; (b) measuring a cellular phenotype of the co-cultured differentiated cells of the first or second population; and (c) identifying the test agent as a modulatory agent if the cellular phenotype is reduced or increased in the presence of the test agent, wherein the first human subject is identified as having, or predisposed to a neurodegenerative disorder.

Disclosed herein is a method of identifying a modulatory agent, the method comprising: (a) co-culturing a first and a second population of differentiated cells in the presence or absence of a test agent, wherein the first population comprises differentiated cells derived from induced pluripotent stem cells (iPSCs) of a first human subject and wherein the second population comprises differentiated cells derived from a second human subject or from a non-human mammalian subject, wherein the differentiated cells in at least one of said populations comprise pancreatic beta cells, and (b) determining that the test agent is a modulatory agent if a cellular phenotype of the co-cultured differentiated cells of the first or second population is reduced or increased in the presence of the test agent, wherein the first or second human subject is identified as having, or predisposed to diabetes or obesity. In some examples of thisdisclosure, the second population comprises differentiated cells derived from induced pluripotent stem cells from the second human subject. In another example of this disclosure, the second population does not comprise cells differentiated from human embryonic stem cells. In anotherexample ofthis disclosure, the first human subject is identified as having, or predisposed to diabetes or obesity. In yet another example, the second human subject is identified as having, or predisposed to diabetes or obesity. In some examples, in the co-cultured first and second populations the ratio of the differentiated cells of one cell type from the first population to differentiated cells of the one cell type from the second population is greater than 1:1, 10:1, 100:1, 1000:1, or 10,000:1. In other examples, in the co-cultured first and second populations the ratio of the differentiated cells of one cell type from the first population to differentiated cells of the one cell type from the second population is less than 1:1, 10:1, 100:1, 1000:1, or 10,000:1.

In some examples of this disclosure, the disorder is diabetes. In some examples, at least one of the populations is detectably labeled. In some examples, both of the populations are detectably labeled. In some examples, at least one population comprises cells detectably labeled by expression of a fluorescent protein, staining with a fluorescent dye, staining with a fluorescently labeled antibody or staining with a fluorescently labeled protein with high affinity for a cell surface protein or receptor. In some examples of this disclosure, the method further comprises co-culturing a third population of cells comprising differentiated cells of a cell type enriched relative to the first and second populations. In some examples, one of the first and second populations is substantially enriched in a cell type relative to the other population. In some examples, the cell type is a pancreatic beta cell, adipocyte or skeletal muscle cell. In some examples, the cellular phenotype is insulin-mediated uptake of glucose. In some examples, the disorder is obesity. In some examples, the disorder is diabetes and the diabetes is Diabetes Type II. In some examples, either: (a) the differentiated cells in the first population of cells comprise adipocytes and the differentiated cells in the second population of cells comprise pancreatic beta cells or (b) the differentiated cells in the first population of cells comprise pancreatic beta cells and the differentiated cells in the second population of cells comprise adipocytes. In some examples: (a) the differentiated cells in the first population of cells comprise skeletal muscle cells and the differentiated cells in the second population of cells comprise pancreatic beta cells or (b) the differentiated cells in the first population of cells comprise pancreatic beta cells and the differentiated cells in the second population of cells comprise skeletal muscle cells.

Disclosed herein is a method of identifying a modulatory agent, the method comprising: (a) co-culturing a first and a second population of differentiated cells in the presence or absence of a test agent, wherein the first population comprises differentiated cells derived from induced pluripotent stem cells (iPSCs) of a first human subject and wherein the second population comprises differentiated cells derived from a second human subject or from a non-human mammalian subject, wherein the differentiated cells in at least one of said populations comprise neurons, neural stem cells, or neural progenitors; (b) measuring a cellular phenotype of the co-cultured differentiated cells of the first or second population; and (c) identifying the test agent as a modulatory agent if the cellular phenotype is reduced or increased in the presence of the test agent, wherein the first or second human subject is identified as having, or predisposed to diabetes or obesity.

In another aspect, this invention provides a co-culture composition comprising a first and a second population of differentiated cells, wherein the first population comprises differentiated cells derived from induced pluripotent stem cells (iPSCs) derived from a first human subject, wherein the second population comprises differentiated cells derived from a second human subject, the differentiated cells in at least one of said populations comprise neurons, neural stem cells, or neural progenitors, the first or second human subject is identified as having, or predisposed to a neurodegenerative disorder. In some embodiments, the second population comprises differentiated cells derived from induced pluripotent stem cells from the second human subject. In some embodiments, the second population does not comprise cells differentiated from human embryonic stem cells. In another embodiment, the neurodegenerative disorder does not comprise ALS caused by a SOD^{G37R} mutation.. In some embodiments of this aspect, the neurodegenerative disorder does not comprise ALS caused by a SOD1^{G93A}, SOD1^{G85R}, or SOD1^{G37R} mutation. In yet another embodiment, the first human subject is identified as having, or predisposed to a neurodegenerative disorder. In a further embodiment, the second human subject is identified as having, or predisposed to a neurodegenerative disorder. In some embodiments, in the co-cultured first and second populations the ratio of the differentiated cells of one cell type from the first population to differentiated cells of the one cell type from the second population is greater than 1:1, 10:1, 100:1, 1000:1, or 10,000:1. In another embodiment, in the co-cultured first and second populations the ratio of the differentiated cells of one cell type from the first population to differentiated cells of the one cell type from the second population is less than 1:1, 10:1, 100:1, 1000:1, or 10,000:1. In another embodiment, the neurodegenerative disorder is a sporadic form of a neurodegenerative disorder. In another embodiment, at least one of the populations is detectably labeled. In yet another embodiment, both of the populations are detectably labeled.

In yet another embodiment of this aspect, the at least one population comprises cells detectably labeled by expression of a fluorescent protein, staining with a fluorescent dye, staining with a fluorescently labeled antibody or staining with a fluorescently labeled protein with high affinity for a cell surface protein or receptor. In a further embodiment, the co-culture composition further comprises a third population of cells comprising differentiated cells of a cell type enriched relative to the first and second populations. In yet another embodiment, the differentiated cells comprise myocytes fused to form myotubes. In yet another embodiment, the cellular phenotype is a synaptic phenotype, an axonal morphology phenotype, or a dendritic morphology phenotype.

In yet another embodiment of this aspect, one of the first and second populations is substantially enriched in a cell type relative to the other population. In still another embodiment, the cell type is an astrocyte, an oligodendrocyte, a motor neuron, a dopaminergic neuron, a cholinergic neuron, a glutamatergic neuron, a serotonergic neuron, a GABAergic interneuron, a myocyte, or a cardiomyocyte. In yet another embodiment, the cellular phenotype is one or more phenotypes selected from the group consisting of: survival, apoptosis, necrosis, axonal degeneration, axonal guidance, axonal morphology, dendritic morphology, receptor density, synaptogenesis, neurogenesis, synapse density, synaptic transmission, synaptic signaling, receptor trafficking, protein trafficking, protein aggregation, proteasome activity, receptor expression, oxidative stress (ROS), FGFR-signaling, FGF signaling, mitochondrial activity, mitochondrial distribution, mitochondrial morphology, and mRNA expression profile. In yet another embodiment, the differentiated cells in the first and second populations of cells comprise neurons. In still another embodiment, the differentiated cells in the first and second populations of cells comprise motor neurons. In yet another embodiment, the neurodegenerative disorder is Amyotrophic Lateral Sclerosis (ALS) or Spinal Muscular Atrophy (SMA). In still another embodiment, the neurodegenerative disorder is ALS and the ALS is a sporadic form of ALS. In a further embodiment, the composition further comprises muscle tissue derived from a healthy subject. In another embodiment, either: (a) the differentiated cells in the first population of cells comprise GABAergic neurons and the differentiated cells in the second population of cells comprise dopaminergic neurons or (b) the differentiated cells in the first population of cells comprise dopaminergic neurons and the differentiated cells in the second population of cells comprise GABAergic neurons. In another embodiment, the neurodegenerative disorder is Parkinson's Disease. In another embodiment, such disorder is a sporadic form of Parkinson's disease. In still another embodiment, such disorder is a familial form of Parkinson's disease. In yet another embodiment either: (a) the differentiated cells in the first population of cells comprise motor neurons and the differentiated cells in the second population of cells comprise astrocytes or (b) the differentiated cells in the first population of cells comprise astrocytes and the differentiated cells in the second population of cells comprise motor neurons. In another embodiment, the neurodegenerative disorder is Huntington's Disease or Amyotrophic Lateral Sclerosis (ALS).

In another embodiment of this aspect either: (a) the differentiated cells in the first population of cells comprise cortical projection neurons (CPNs) and the differentiated cells in the second population of cells comprise spinal cord motor neurons (SCMNs) or (b) the differentiated cells in the first population of cells comprise SCMNs and the differentiated cells in the second population of cells comprise CPNs. In yet another embodiment, the differentiated cells in the first population comprise neurons selected from the group consisting of: motor neurons, dopaminergic neurons, GABAergic neurons, cortical projection neurons, striatal neurons, and spinal cord motor neurons. In yet another embodiment, the differentiated cells in the second population comprise neurons selected from the group consisting of: motor neurons, dopaminergic neurons, GABAergic neurons, cortical projection neurons, striatal neurons, and spinal cord motor neurons. In yet another embodiment, the neurodegenerative disorder is selected from the group consisting of: Amyotrophic Lateral Sclerosis (ALS), Spinal Muscular Atrophy (SMA), Huntington's Disease, and Parkinson's Disease. In yet another embodiment, the neurodegenerative disorder is selected from the group consisting of: Alexander's disease, Alper's disease, Alzheimer's disease, amyotrophic lateral sclerosis, ataxia telangiectasia, Batten disease, bovine spongiform encephalopathy, Canavan disease, Cockayne syndrome, corticobasal degeneration, Creutzfeldt-Jakob disease, Huntington's disease, HIV-associated dementia, Kennedy's disease, Krabbe's disease, lewy body dementia, Machado-Joseph disease, multiple sclerosis, multiple system atrophy, narcolepsy, neuroborreliosis, Parkinson's disease, Pelizaeus-Merzbacher Disease, Pick's disease, primary lateral sclerosis, prion diseases, Refsum's disease, Sandhoffs disease, Schilder's disease, subacute combined degeneration of spinal cord secondary to pernicious anaemia, schizophrenia, spinocerebellar ataxia, spinal muscular atrophy, Steele-Richardson-Olszewski disease, and tabes dorsalis.

Disclosed herein is a co-culture composition comprising a first and a second population of differentiated cells, wherein the first population comprises differentiated cells derived from induced pluripotent stem cells (iPSCs) derived from a first human subject, wherein the second population comprises differentiated cells derived from a second human subject or from a non-human mammalian subject, the differentiated cells in at least one of said populations comprise pancreatic beta cells, the first or second human subject is identified as having, or predisposed to diabetes or obesity. In some examples of this disclosure, the second population comprises differentiated cells derived from induced pluripotent stem cells from the second human subject. In some examples of this disclosure, the second population does not comprise cells differentiated from human embryonic stem cells. In some examples of this disclosure, the first human subject is identified as having, or predisposed to diabetes or obesity. In some examples of this disclosure, the second human subject is identified as having, or predisposed to diabetes or obesity. In some examples of this disclosure, in the co-cultured first and second populations the ratio of the differentiated cells of one cell type from the first population to differentiated cells of the one cell type from the second population is greater than 1:1, 10:1, 100:1, 1000:1, or 10,000:1. In some examples of this disclosure, in the co-cultured first and second populations the ratio of the differentiated cells of one cell type from the first population to differentiated cells of the one cell type from the second population is less than 1:1, 10:1, 100:1, 1000:1, or 10,000:1. In some examples, the disorder is diabetes. In other examples, at least one of the populations is detectably labeled. In some examples, both of the populations are detectably labeled. In some examples, at least one population comprises cells detectably labeled by expression of a fluorescent protein, staining with a fluorescent dye, staining with a fluorescently labeled antibody or staining with a fluorescently labeled protein with high affinity for a cell surface protein or receptor. In some examples, the composition further comprises a third population of cells comprising differentiated cells of a cell type enriched relative to the first and second populations. In some examples, one of the first and second populations is substantially enriched in a cell type relative to the other population.

In some examples of this disclosure, the cell type is a pancreatic beta cell, adipocyte or skeletal muscle cell. In yet other examples, the cellular phenotype is insulin-mediated uptake of glucose. In other examples, the disorder is obesity. In still other examples the disorder is diabetes and the diabetes is Diabetes Type II. In yet other examples, either: (a) the differentiated cells in the first population of cells comprise adipocytes and the differentiated cells in the second population of cells comprise pancreatic beta cells or (b) the differentiated cells in the first population of cells comprise pancreatic beta cells and the differentiated cells in the second population of cells comprise adipocytes. In some examples, either: (a) the differentiated cells in the first population of cells comprise skeletal muscle cells and the differentiated cells in the second population of cells comprise pancreatic beta cells or (b) the differentiated cells in the first population of cells comprise pancreatic beta cells and the differentiated cells in the second population of cells comprise skeletal muscle cells.

Also disclosed, is a method of identifying a cytoprotective agent, the method including: co-culturing differentiated cells derived from induced stem cells (iSCs) or iPS cells that are two or more differentiated cell types from a first human subject and a second human subject in the presence or absence of a test agent; and indicating that the test agent is a cytoprotective agent if survival of the co-cultured differentiated cells from the first subject is greater in the presence of the test agent or indicating that the test agent is not a cytoprotective agent if the survival in the presence of the test agent is the same or less than in the absence of the test agent, wherein the first human subject is normal and the second human subject is identified as suffering from or prediposed to a disorder. In some examples, the disorder of the herein-provided methods is one of stroke, cognitive impairment, and a mood disorder.

Also disclosed herein, is a method of identifying a neuroprotective agent, the method including: co-culturing differentiated cells derived from iSCs or iPS cells that are two or more differentiated cell types from a first human subject and a second human subject in the presence or absence of a test agent; and indicating that the test agent is a neuroprotective agent if survival of the co-cultured differentiated cells from the first subject is greater in the presence of the test agent or indicating that the test agent is not a neuroprotective agent if the survival in the presence of the test agent is the same or less than in the absence of the test agent, wherein the first human subject is normal and the second human subject is identified as suffering from or predisposed to a neurodegenerative disease or condition.

In some examples of this disclosure, the neurodegenerative disease of the herein-provided methods is selected from the group consisting of Alexander's disease, Alper's disease, Alzheimer's disease, amyotrophic lateral sclerosis, ataxia telangiectasia, Batten disease, bovine spongiform encephalopathy, Canavan disease, Cockayne syndrome, corticobasal degeneration, Creutzfeldt-Jakob disease, Huntington's disease, HIV-associated dementia, Kennedy's disease, Krabbe's disease, lewy body dementia, Machado-Joseph disease, multiple sclerosis, multiple system atrophy, narcolepsy, neuroborreliosis, Parkinson's disease, Pelizaeus-Merzbacher Disease, Pick's disease, primary lateral sclerosis, prion diseases, Refsum's disease, Sandhoffs disease, Schilder's disease, subacute combined degeneration of spinal cord secondary to pernicious anaemia, schizophrenia, spinocerebellar ataxia, spinal muscular atrophy, Steele-Richardson-Olszewski disease, and tabes dorsalis.

In some examples, one or more of the differentiated cells are induced to express SOD1^{G93A}, SOD1^{G85R}, or SOD1^{G37R}. In some examples of this disclosure, the neurodegenerative disorder does not comprise ALS caused by a SOD1^{G93A}, SOD1^{G85R}, or SOD1^{G37R} mutation. In some disclosures, the first and second differentiated cell types comprise neurons, motor neurons, dopaminergic neurons, or glial cells.

Also disclosed herein, in another aspect, is a method of identifying a neuroprotective agent, the method including: culturing a first differentiated cell type derived from iSCs or iPS cells in the presence of conditioned medium from a second differentiated cell type derived from iSCs iPS cells; and indicating that the test agent is a neuroprotective agent if survival of the first differentiated cell type is greater in the presence of the test agent or indicating that the test agent is not a neuroprotective agent if the survival in the presence of the test agent is the same or less than in the absence of the test agent, wherein the first differentiated cell type is normal and the second differentiated cell type is identified as suffering from or predisposed to a neurodegenerative condition.

Additionally disclosed herein is a method of identifying a neuroprotective agent, the method including culturing a first differentiated cell type derived from in the presence or absence of (i) a second differentiated cell type derived from iSCs iPS cells; or (ii) conditioned medium from differentiated cells of the second differentiated cell type; wherein the differentiated cells of the first and second differentiated cell types are from a human subject identified as suffering from or predisposed to a neurodegenerative disease.

Also disclosed is an iSC-derived (or iPS-derived) cell expressing any of SOD 1^{G93A}, SOD1^{G85R}, or SOD1^{G37R}.

### DETAILED DESCRIPTION OF THE INVENTION

### I. Introduction

There is a need in the art for *in vitro* models that closely recapitulate the cellular interplay that contributes to disease. Such disease models can be used to identify the causative agents of disease and to, potentially, screen for therapeutic compounds. The basis for many of the disease models presented herein are cells differentiated from induced pluripotent stem cells (iPS) derived from healthy subjects or subjects suffering from various diseases. For example, some of the models provided herein rely on differentiated cells from iPS cells derived from a human subject with a sporadic neurodegenerative condition (e.g., ALS). The disease models also combine different cell-types (e.g., a neurons with an astrocyte), combine cells from different human subjects (e.g., a healthy human subject and a diseased human), and/or combine cells from human subjects with cells from non-human subjects (e.g., mice).

The disease models provided herein have many possible uses. Importantly, they can be used to illuminate the mechanisms underlying disease. For example, they can help identify: (1) whether cell non-autonomous or cell autonomous factors contribute to a specific disease or disorder; (2) which cell-types cause a specific disease or disorder; (3) which cell-types are affected by a disease or disorder; and (4) which component of a cell is contributing to a specific disease or disorder, e.g., they can help establish a synaptic locus (pre- versus post-) of a neurodegenerative disease. The disease models provided herein can also be adopted for high-throughput screening assays to identify lead therapeutic agents to treat a specific disease or disorder, or otherwise correct a phenotype.

### II. Some Definitions

For convenience, induced multipotent and pluripotent stem cell lines are referred to as "induced stem cell lines" (iSC lines) herein. Induced pluripotent stem cells are referred to as iPS cells or iPSCs.

"Correcting" a phenotype, as used herein, refers to altering a phenotype such that it more closely approximates a normal phenotype.

"iSC donor" as used herein, refers to a subject, e.g., a human patient from which one or more induced pluripotent stem cell lines have been generated. Generally, the genome of an iSC line corresponds to that of its donor. Similarly, "iPSC donor" as used herein, refers to a subject, e.g., a human patient from which one or more induced pluripotent stem cell lines have been generated. Generally, the genome of an iPSC line corresponds to that of its donor.

"Phenomic analysis," as used herein, refers to the analysis of phenotypes (e.g., resting calcium level, gene expression profiles, apoptotic index, electrophysiological properties, sensitivity to free radicals, compound uptake and extrusion, kinase activity, second messenger pathway responses) exhibited by a particular type of cell (e.g., cardiomyocytes).

"Phenome," as used herein refers to the set of phenotypes that is subject and cell-type specific. For example, the phenome of hepatocytes and cardiomyocytes from the same individual will be quite distinct even though they share the same genome.

An "endogenous allele," as used herein, refers to a naturally occurring allele that is native to the genome of a cell, i.e., an allele that is not introduced by recombinant methodologies.

A "disease allele," as used herein, refers to a gene variant - naturally occurring or introduced by recombinant methodologies - that contributes to the onset of a disease.

A "normal" phenotype, as used herein, refers to a phenotype (e.g., apoptotic rate, resting calcium level, kinase activity, gene expression level) that falls within a range of phenotypes found in healthy individuals or that are not associated with (e.g., predictive of) a health condition, disease or disorder.

A "wild-type" subject or cell as used herein refers to a subject or cell that is considered to be free from any known mutations or disease alleles.

The term "derived" refers to the ordinary usage and meaning of the term, and includes, but is in no way limited to, the process wherein a cell (e.g., neuron, pancreatic beta cell, adipocyte, etc.) is differentiated from an iPSC. An "iPS-derived cell" as used herein, refers to a cell that is generated from an iPSC either by proliferation of the iPSC, or by differentiation of the iPSC into a different cell type. iPSC-derived cells include cells not differentiated directly from an iPSC, but from an intermediary cell type, e.g., a glial progenitor cell, a neural stem cell, or a cardiac progenitor cell. Similarly, an "iSC-derived cell" as used herein, refers to a cell that is generated from an iSC either by proliferation of the iSC, or by differentiation of the iSC into a different cell type. iSC-derived cells include cells not differentiated directly from an iSC, but from an intermediary cell type, e.g., a glial progenitor cell, a neural stem cell, or a cardiac progenitor cell.

### III. Co-Cultures to Model Disease

### A. Co-cultures

Provided herein are methods of co-culturing cells, as well as compositions of co-cultured cells. In some embodiments, healthy cells are co-cultured with cells derived from a subject who has, or is predisposed to, a neurodegenerative disorder (e.g., neurodegenerative disease, ALS, sporadic ALS, Parkinson's Disease (PD), Spinal Muscular Atrophy (SMA), Huntington's Disease (HD), diabetes). The healthy cells may be derived from a subject who is "healthy" in that the subject does not have a disorder under study and/or is not predisposed to having such disorder. The healthy cells may be co-cultured with cells derived from a subject who is not "healthy" in that the subject either has a neurodegenerative disorder (e.g., neurodegenerative disease, ALS, PD, SMA, HD), or is predisposed to having such a disorder. In some cases, one or more cells or cell-types are differentiated from induced pluripotent stem cells (iPS) derived from a subject. Cells differentiated from iPS may also be co-cultured with cells or tissue (e.g., muscle tissue, skeletal muscle tissue, adipocytes) extracted from a subject (e.g., human, mouse). In some cases, cells differentiated from iPS cells derived from a healthy subject (e.g., human, mouse) are co-cultured with cells differentiated from iPS cells derived from a subject (e.g., human, mouse) having, or predisposed to a neurodegenerative disorder (e.g., neurodegenerative disease, ALS, sporadic ALS, Parkinson's Disease (PD), Spinal Muscular Atrophy (SMA), Huntington's Disease (HD), diabetes, Alzheimer's Disease). In some examples disclosed herein, cells from one species (e.g., iPS cells from human) are co-cultured with cells of a different species (e.g., mouse ES cells). In the present invention, cells from the same species (human with human) are co-cultured together. In some cases, individuals predisposed to a a disease are identified as predisposed to the disease base on the presence of a polymorphic allele associated with the disease in a genome wide association (GWAS) study. Lists of publicly available databases containing alleles associated with a health condition are provided in Table 1. Disease-associated polymorphisms have been identified for a number of neurodegenerative and metabolic disorders. In some cases, iPSCs are derived from subjects that have been diagnosed with a disease and also have a polymorphic allele associated with the disease. In other cases, iPSCs are derived from subjects that have a disease-associated polymorphic allele, but, as of yet, have not been diagnosed with the corresponding disease.

| **Table 1** | | |
|---|---|---|
| **List of Publicly Available Databases Containing Alleles Associated with a Health Condition or Predisposition to a Health Condition** | | |
| **Name of Website** | **Website URL** | **Brief Description** |
| Alzgene | www.alzforum.org/res/com/gen/ alzgene | Collection of published genetic association studies performed on Alzheimer Disease phenotypes, from database searches and journals' contents lists. Case and control data presented. |
| Cytokine Gene Polymorphism in Human Disease | www.nanea.dk/cytokinesnps/ | Regularly updated database with Medline-based records from a systematic review of cytokine gene polymorphisms associated with human disease. Data extracted from two publications about the study. |
| HuGE Navigator | hugenavigator.net | HuGE Navigator provides access to a continuously updated knowledge base in human genome epidemiology, including information on population prevalence of genetic variants, gene-disease associations, gene-gene and gene-environment interactions, and evaluation of genetic tests. |
| GenAtlas | www.genatlas.org | Regularly updated database of genes, phenotypes and references. Among numerous databases are brief sections on disorders associated with genes, with lists of citations. May be biased towards statistically significant results. |
| GeneCanvas | genecanvas.idf.inserm.fr | Database of cardiovascular candidate genes and their polymorphisms investigated at INSERM (Paris, France). Data include gene frequencies and linkage disequilibrium statistics. |
| Genetic Association Database | geneticassociationdb.nih.gov | Database of human genetic association studies of complex diseases and disorders, based on Medline records. Data extracted from publications. |
| Human Obesity Gene Map Database | obesitygene.pbrc.edu | Database of obesity-related genes, including P values for association and references. Biased in favour of statistically significant results. |
| Infevers | fmf.igh.cnrs.fr/infevers | Database of genetic associations in hereditary inflammatory disorders, with voluntarily submitted entries. Submissions are validated by an editorial board member. |
| MedGene | medgene.med.harvard.edu/MEDGENE/ | Automated database of gene disease association studies in Medline. |
| OMIM | www.ncbi.nlm.nih.gov/omim/ | Database of human genes and genetic disorders, containing textual information with links to Medline and sequence records in the Entrez system, and links to additional related resources at NCBI and elsewhere. |
| PharmGKB | www.pharmgkb.org | Database of genomic data and clinical information from participants in pharmacogenetics research studies. Welcomes submission of primary data. |
| T1DBase | t1dbase.org/ | Database of type 1 diabetes data, including information from collaborating laboratories. Some indication given of unpublished data. |

Different combinations of neurons can be co-cultured together. In some cases, neurons of the same cell-type from two different subjects, may be co-cultured together. For example, motor neurons (MNs) derived from a healthy subject (human) can be co-cultured with MNs derived from induced pluripotent stem cells (iPS) derived from a human subject with a neurodegenerative disease (e.g., ALS, SMA). In addition, MNs derived from a healthy subject can be co-cultured with MNs derived from iPS derived from a human subject with a neurodegenerative disease (e.g., ALS, SMA) along with muscle tissue from a subject (e.g., a healthy human subject). In some cases, MNs derived from a healthy subject can be co-cultured with MNs derived from iPS derived from a human subject having or predisposed to ALS with a known genetic component (e.g., a defect in SOD1, TDP-43, fus, chr 9). Or, MNs derived from a healthy subject can be co-cultured with MNs derived from iPS derived from a human subject having or predisposed to ALS with a known genetic component (e.g., SOD1, TDP-43, fus, chr 9) and with muscle tissue from the same or different subject (e.g., healthy human subject).

The cells are derived from different subjects. Non-limiting examples include the following: MNs derived from a healthy subject can be co-cultured with glial cells (e.g., astrocytes) derived from a human subject having or predisposed to a neurodegenerative disease (e.g., ALS, SMA) along with muscle tissue from a subject (e.g., a healthy human subject); MNs derived from a subject having or predisposed to a neurodegenerative disease (e.g., ALS, SMA) can be co-cultured with glial cells (e.g., astrocytes) derived from a healthy subject along with muscle tissue from a subject (e.g., a healthy human subject); dopaminergic neurons derived from a subject having or predisposed to a neurodegenerative disease (e.g., PD) can be co-cultured with GABAergic neurons derived from a healthy subject; GABAergic neurons derived from a subject having or predisposed to a neurodegenerative disease (e.g., PD) can be co-cultured with dopaminergic neurons derived from a healthy subject; cortical projection neurons from a healthy subject can be co-cultured with spinal cord MN from a subject having or predisposed to a neurodegenerative disease (e.g., ALS); cortical projection neurons (CPN) from a subject having or predisposed to a neurodegenerative disease (e.g., ALS) can be co-cultured with spinal cord MNs (SCMNs) from a healthy subject. In some examples, neural cells (e.g., glial cells, astrocytes, MNs, and/or SCMNs) derived from a healthy subject can be co-cultured with , neural cells (e.g., glial cells, astrocytes, MNs, and/or SCMNs) derived from iPS derived from a human subject having or predisposed to ALS with a known genetic component (e.g., SOD1, TDP-43, fus, chr 9). In some cases, pyramidal neurons derived from a subject suffering from Alzheimer's disease are co-cultured with pyramidal neurons derived from healthy individuals (e.g., healthy individuals from about 40 to about 90 years of age) for a period of time generally sufficient to allow formation of synapses, e.g., at least about 4 weeks to about 10 weeks..

As disclosed herein, different combinations of cells can also be co-cultured in order to model metabolic disease such as diabetes (e.g., Diabetes Type II (DTII) or Diabetes Type I (DTI)). Pancreatic beta cells differentiated from iPS from a healthy human subject maybe co-cultured with adipocytes derived from one or more of the following human subjects: (1) healthy non-obese human subjects; (2) obese with DTII human subjects; (3) obese without DTII human subjects; (4) non-obese with DTII human subjects; (5) non-obese human subjects at risk for DTII or obesity; (6) non-obese human subjects at risk for DTII; (7) DTII human subjects at risk for obesity; (8) obese with DTI human subjects; (9) obese without DTI human subjects; (10) non-obese with DTI human subjects; (11) non-obese human subjects at risk for DTI or obesity; (12) non-obese human subjects at risk for DTI; and/or (13) DTI human subjects at risk for obesity. Pancreatic beta cells differentiated from iPS from a healthy human subject can also be co-cultured with skeletal muscle cells derived from one or more of the following human subjects: (1) healthy non-obese human subjects; (2) obese with DTII human subjects; (3) obese without DTII human subjects; (4) non-obese with DTII human subjects; (5) non-obese human subjects at risk for DTII or obesity; (6) non-obese human subjects at risk for DTII; (7) DTII human subjects at risk for obesity; (8) obese with DTI human subjects; (9) obese without DTI human subjects; (10) non-obese with DTI human subjects; (11) non-obese human subjects at risk for DTI or obesity; (12) non-obese human subjects at risk for DTI; and/or (13) DTI human subjects at risk for obesity.. Adipocytes differentiated from iPS from a healthy human subject can be co-cultured with pancreatic beta cells derived from one or more of the following human subjects: (1) healthy non-obese human subjects; (2) obese with DTII human subjects; (3) obese without DTII human subjects; (4) non-obese with DTII human subjects; (5) non-obese human subjects at risk for DTII; (6) non-obese human subjects at risk for DTII; (7) DTII human subjects at risk for obesity; (8) obese with DTI human subjects; (9) obese without DTI human subjects; (10) non-obese with DTI human subjects; (11) non-obese human subjects at risk for DTI or obesity; (12) non-obese human subjects at risk for DTI; and/or (13) DTI human subjects at risk for obesity. Skeletal muscle cells differentiated from iPS from a healthy human subject can be co-cultured with pancreatic beta cells derived from one or more of the following human subjects: (1) healthy non-obese human subjects; (2) obese with DTII human subjects; (3) obese without DTII human subjects; (4) non-obese with DTII human subjects; (5) non-obese human subjects at risk for DTII; (6) non-obese human subjects at risk for DTII; (7) DTII human subjects at risk for obesity; (8) obese with DTI human subjects; (9) obese without DTI human subjects; (10) non-obese with DTI human subjects; (11) non-obese human subjects at risk for DTI or obesity; (12) non-obese human subjects at risk for DTI; and/or (13) DTI human subjects at risk for obesity..

The ratio of differentiated cells within a co-culture population of cells may vary. The ratio of differentiated cells from one subject to differentiated cells from another subject may be greater than or equal to: 10,000:1, 5,000:1, 2,500:1, 1,000:1; 750:1; 500:1, 250:1, 100:1, 50:1, 25:1, 10:1, 5:1, 4:1, 3:1, 2:1, or 1:1. The ratio of differentiated cells may be obtained naturally, by co-culturing conditions, by purification (e.g., FACS sorting), or other method known in the art.

The co-cultures may comprise 1, 2, 3, 4, 5, 6, 7, 8, 9, 10, 15, 20, greater than about 1, greater than about 2, greater than about 5, greater than about 10, or greater than about 15 different cell-types. Non-limiting examples of cell-types include: neural cells, neural progenitor cells, neurons, glial cells, astrocytes, motor neurons (MNs), dopaminergic neurons, spinal cord MNs, cortical projection neurons (CPNs), skeletal neurons, striatal neurons, GABAergic neurons, pancreatic beta cells, adipocytes, skeletal muscle cells, myocytes, oligodendrocytes, cholinergic neurons, pancreatic progenitor cells, insulin producing cells, glutamatergic neurons, serotonergic neurons, or cardiomyocytes.

The co-cultures may also include cells detectably labeled in order to identify the cell-type (e.g., MN vs. astrocyte). The labeling may occur by genetically modifying iPS cells to express a reporter gene (e.g., GFP, mCherry, YFP, other fluorescent marker, beta lactamase, Fluorescent Timer protein, beta galactosidase, beta glucuronidase, etc..) under the control of a promoter for a marker of a cell-type fate (e.g., MN, astrocyte, endoderm, etc.), as described herein. In some cases, greater than one reporter construct is used in order to identify different fates (e.g., red for MNs, green for astrocytes). The reporter is then expressed upon differentiation of the iPS into the appropriate cell-type. Different cell types may also be labeled by using an antibody or ligand directed to a cell-surface markers (e.g., CXCR4, CKIT for endoderm) associated with a specific cell-fate. Similarly, different cell types may also be labeled by using an antibody or ligand directed to an intracellular marker associated with a specific cell-fate. In some cases, the cells are FACS-sorted in order to obtain purified populations of cells of a specific cell-type. The same cell-types (e.g., motor neurons) but from two or more different sources (e.g., healthy subject, diseased subject), may be pre-labeled with a marker (e.g., fluorescent marker) that is non-specific but that will suffice to identify the cell-type when mixed with cells from a different source. For example, MNs derived from a healthy subject may be labeled with GFP in a non-cell-type specific manner, for example by GFP-tagged antibody to any common cell-surface marker, and combined with MNs derived from a diseased subject that has been pre-labeled in a similar manner, but to express a different marker, e.g., mCherry, RFP. FACS sorting may also be helpful in order to generate ratios of cell-types, as described herein.

The co-cultures provided herein enable the analysis of a variety of phenotypes. Non-limiting examples of phenotypes that can be observed or monitored are: cell survival, apoptosis, necrosis, axonal degeneration, axonal guidance, axonal morphology, dendritic morphology, receptor density, synaptogenesis, neurogenesis, synapse density, synaptic transmission, synaptic signaling, receptor trafficking, protein trafficking, protein aggregation, proteasome activity, receptor expression, oxidative stress (ROS), FGFR-signaling, FGF signaling, mitochondrial activity, mitochondrial potential, mitochondrial distribution, mitochondrial morphology, insulin-mediated glucose uptake, and mRNA expression profile.

The co-cultures provided herein may enable the determination of whether a phenotype is cell-autonomous or cell-non-autonomous. For example, if healthy MNs demonstrate an impaired phenotype when co-cultured with diseased astrocytes, but not healthy astrocytes, that may suggest that the phenotype is cell-non-autonomous. The co-cultures provided herein may also help determine which cell-type (e.g., astrocyte, MN, etc.) is causing the phenotype.

### B. Subjects

Some of the methods described herein utilize differentiated cells (or panels of differentiated cells) from induced stem cells or iPS derived from subjects that meet one or more pre-determined criteria. In some cases subjects and cellular samples from such subjects may be selected for the generation of induced stem cell lines and panels of induced stem cell lines based on one or more of such pre-determined criteria. These include, but are not limited to, the presence or absence of a health condition in a subject, one or more positive diagnostic criteria for a health condition, a family medical history indicating a predisposition or recurrence of a health condition, the presence or absence of a genotype associated with a health condition, or the presence of at least one polymorphic allele that is not already represented in a panel of induced stem cell lines. The subject may have a sporadic disease or disorder (e.g., sporadic ALS), a non-genetic disease or disorder, a multifactorial disease or disorder, and/or a genetic disease or disorder.

In some cases, a panel of differentiated cells from induced stem cell lines is generated specifically from individuals diagnosed with a health condition, and from subjects that are free of the health condition.

A co-culture of differentiated cells of the present invention include a combination of one or more of: cells derived from a subject considered to be free of any health condition or predisposition to any health condition, cells derived from a subject considered suffering from a health condition or predisposed to suffer from a health condition, or cells from a subject considered to be free from and not predisposed to suffering from any health condition in which one or more disease associated alleles or genetic defects have been introduced. Such disease associated alleles may include but are not limited to alleles of SOD1 including for example the G93A, G85R, and G37R alleles; alleles of APOE including for example the epsilon 4 allele; alleles of NPC1; alleles of the Huntington's disease gene; alleles of SCA1, SCA2, and SCA3; alleles of DRPLA; and alleles of the androgen receptor gene. In addition, to model Spinal Muscular Atrophy, defects in the *Survival Motor Neuron* (SMN) gene (e.g., SMN1 or SMN2), can be introduced. And, to model Parkinson's Disease, mutations in the genes that encode α-synuclein (PARK1), parkin (PARK2), PINK 1 (PARK6), or LRRK2 (PARK8) can be introduced. Similarly, the differentiated cells can be derived from a subjected identified as having specific allelic variations (e.g., SOD1 including for example the G93A, G85R, and G37R alleles; alleles of APOE including for example the epsilon 4 allele; alleles of NPC1; alleles of the Huntington's disease gene; alleles of SCA1, SCA2, and SCA3; alleles of DRPLA; and alleles of the androgen receptor gene) and/or other genetic defects or mutations in disease-associated genes (e.g., SMN, MN1, SMN2, PARK1, PARK2, PARK6, PARK8, SOD1, APOE, SCA1, SCA2, SCA3, etc.)

As disclosed herein such health conditions (e.g., diseases or disorders) include neurodegenerative disorders; neurological disorders such as cognitive impairment, and mood disorders; deafness; osteoporosis; cardiovascular diseases; diabetes; metabolic disorders; respiratory diseases; drug sensitivity conditions; eye diseases; immunological disorders; hematological diseases; kidney diseases; proliferative disorders; genetic disorders, traumatic injury, stroke, metabolic disorders, or organ failure. According to the present invention, the health condition is a neurodegenerative disorder or disease. In other disclosures herein, the health condition is a metabolic disorder (e.g., diabetes, Diabetes Type II (DTII), Diabetes Type I (DTI)). In some disclosures herein, the health condition is obesity. In some disclosures herein, the health condition is diabetes, e.g., DTII.

Examples of neurodegenerative disorders or diseases include, but are not limited to, Alexander's disease, Alper's disease, Alzheimer's disease, amyotrophic lateral sclerosis (ALS), ataxia telangiectasia, Batten disease, bovine spongiform encephalopathy, Canavan disease, Cockayne syndrome, corticobasal degeneration, Creutzfeldt-Jakob disease, Huntington's disease, HIV-associated dementia, Kennedy's disease, Krabbe's disease, lewy body dementia, Machado-Joseph disease, multiple sclerosis, multiple system atrophy, narcolepsy, neuroborreliosis, Parkinson's disease, Pelizaeus-Merzbacher Disease, Pick's disease, primary lateral sclerosis, prion diseases, Refsum's disease, Sandhoffs disease, Schilder's disease, subacute combined degeneration of spinal cord secondary to pernicious anaemia, schizophrenia, spinocerebellar ataxia, spinal muscular atrophy, Steele-Richardson-Olszewski disease, and tabes dorsalis.

Examples of neurological disorders include, stroke, cognitive impairment, and mood disorders.

Such subjects may be identified in, e.g., gene association studies, clinical studies, and hospitals, preferably after a final diagnosis of a health condition has been made. Preferably, subjects are identified in gene association studies that include non-affected control individuals.

In other cases, iSC lines or iPS cell lines are generated from subjects screened for the presence or absence of at least one allele associated with a health condition or a predisposition for a health condition. Such alleles indicate that an individual, though not exhibiting overt symptoms of a health condition, has a high risk of developing the health condition. Genotyping of subjects may be performed on samples from a number of sources, e.g., blood banks, sperm banks, gene-association studies, hospitals, clinical trials, or any other source as long as a living cellular sample can be obtained from the individual that is genotyped. While not wishing to be bound by theory, it is believed that one or more cellular phenotypes from individuals carrying alleles associated with health conditions exhibit abnormalities that can serve as more reliable prognostic indicators of a health condition in combination with a genotype than a genotype alone. Further, identification of specific abnormal cellular phenotypes associated with a health condition may indicate a target pathway for screening of prophylactic and therapeutic agents for the health condition.

In still other cases, iSC lines or iPS cell lines are generated from healthy subjects, and the disease or condition to be studied is recapitulated by introducing an allele associated with that disease or condition using recombinant DNA techniques. For example, the SOD1G93A allele may be introduced into iSC cells or iPS cells which may then be differentiated into motor neurons or astrocytes and used for screening for neuroprotective compounds. The disease associated allele may be introduced into an iSC cell line, iPS cell line, or into the differentiated cells derived from an iSC line or iPS cell.

### C. Sources of Cells

The multipotent or pluripotent cells (e.g., iSCs, iPS cells) may be induced from a wide variety of mammalian cells and by any method known in the art. Examples of suitable populations of mammalian cells include those that include, but are not limited to: fibroblasts, bone marrow-derived mononuclear cells, skeletal muscle cells, adipose cells, peripheral blood mononuclear cells, macrophages, hepatocytes, keratinocytes, oral keratinocytes, hair follicle dermal cells, gastric epithelial cells, lung epithelial cells, synovial cells, kidney cells, skin epithelial cells or osteoblasts.

The cells can also originate from many different types of tissue, e.g., bone marrow, skin (e.g., dermis, epidermis), muscle, adipose tissue, peripheral blood, foreskin, skeletal muscle, or smooth muscle. The cells can also be derived from neonatal tissue, including, but not limited to: umbilical cord tissues (e.g., the umbilical cord, cord blood, cord blood vessels), the amnion, the placenta, or other various neonatal tissues (e.g., bone marrow fluid, muscle, adipose tissue, peripheral blood, skin, skeletal muscle etc.).

The cells can be derived from neonatal or post-natal tissue collected from a subject within the period from birth, including cesarean birth, to death. For example, the tissue may be from a subject who is > 10 minutes old, > 1 hour old, > 1 day old, > 1 month old, > 2 months old, > 6 months old, > 1 year old, >2 years old, >5 years old, >10 years old, >15 years old, >18 years old, >25 years old, >35 years old, >45 years old, >55 years old, >65 years old, >80 years old, <80 years old, <70 years old, <60 years old, <50 years old, <40 years old, <30 years old, <20 years old or <10 years old. The subject may be a neonatal infant. In some cases, the subject is a child or an adult. In some examples, the tissue is from a human of age 2, 5, 10 or 20 hours. In other examples, the tissue is from a human of age 1 month, 2 months, 3 months, 4 months, 5 months, 6 months, 9 months or 12 months. In some cases, the tissue is from a human of age 1 year, 2 years, 3 years, 4 years, 5 years, 18 years, 20 years, 21 years, 23 years, 24 years, 25 years, 28 years, 29 years, 31 years, 33 years, 34 years, 35 years, 37 years, 38 years, 40 years, 41 years, 42 years, 43 years, 44 years, 47 years, 51 years, 55 years, 61 years, 63 years, 65 years, 70 years, 77 years, 85 years, or 90 years old.

According to the present invention, the cells are from a human subject. As disclosed herein the cells can also be derived from non-human subjects, e.g., non-human mammals. Examples of non-human mammals include, but are not limited to, non-human primates (e.g., apes, monkeys, gorillas), rodents (e.g., mice, rats), cows, pigs, sheep, horses, dogs, cats, or rabbits.

The cells may be collected from subjects with a variety of disease statuses. The cells can be collected from a subject who is free of an adverse health condition. In other cases, the subject is suffering from, or at high risk of suffering from, a disease or disorder, e.g., a chronic health condition such as cardiovascular disease, eye disease (e.g., macular degeneration), auditory disease, (e.g., deafness), diabetes, cognitive impairment, schizophrenia, depression, bipolar disorder, dementia, neurodegenerative disease, Alzheimer's Disease, Parkinson's Disease, multiple sclerosis, osteoporosis, liver disease, kidney disease, autoimmune disease, arthritis, or a proliferative disorder (e.g., a cancer). In other cases, the subject is suffering from, or at high risk of suffering from, an acute health condition, e.g., stroke, spinal cord injury, burn, or a wound. In certain cases, a subject provides cells for his or her future use (e.g., an autologous therapy), or for the use of another subject who may need treatment or therapy (e.g., an allogeneic therapy). In some cases, the donor and the recipient are immunohistologically compatible or HLA-matched.

The cells to be induced can be obtained from a single cell or a population of cells. The population may be homogeneous or heterogeneous. The cells may be a population of cells found in a human cellular sample, e.g., a biopsy or blood sample. Often, the cells are somatic cells. The cells may be a cell line. In some cases, the cells are derived from cells fused to other cells. In some cases, the cells are not derived from cells fused to other cells. In some cases, the cells are not derived from cells artificially fused to other cells. In some cases, the cells are not a cell that has undergone the procedure known as somatic cell nuclear transfer (SCNT) or a cell descended from a cell that underwent SCNT.

The cellular population may include both differentiated and undifferentiated cells. In some cases, the population primarily contains differentiated cells. In other cases, the population primarily contains undifferentiated cells, e.g., undifferentiated stem cells. The undifferentiated cells within the population may be induced to become pluripotent or multipotent. In some cases, differentiated cells within the cellular population are induced to become pluripotent or multipotent.

### Collection of Cells

Methods for obtaining human somatic cells are well established, as described in, e.g., Schantz and Ng (2004), A Manual for Primary Human Cell Culture, World Scientific Publishing Co., Pte, Ltd. In some cases, the methods include obtaining a cellular sample, e.g., by a biopsy (e.g., a skin sample), blood draw, or alveolar or other pulmonary lavage. It is to be understood that initial plating densities from of cells prepared from a tissue may be varied based on such variable as expected viablility or adherence of cells from that particular tissue. Methods for obtaining various types of human somatic cells include, but are not limited to, the following exemplary methods:

### 1. Postnatal Skin

Skin tissue containing the dermis is harvested, for example, from the back of a knee or buttock. The skin tissue is then incubated for 30 minutes at 37 °C in 0.6% trypsin/Dulbecco's Modified Eagle's Medium (DMEM)/F-12 with 1% antibiotics/antimycotics, with the inner side of the skin facing downward.

After the skin tissue is turned over, tweezers are used to lightly scrub the inner side of the skin. The skin tissue is finely cut into 1 mm² sections using scissors and is then centrifuged at 1200 rpm and room temperature for 10 minutes. The supernatant is removed, and 25 ml of 0.1% trypsin/DMEM/F-12/1% antibiotics, antimycotics, is added to the tissue precipitate. The mixture is stirred at 200-300 rpm using a stirrer at 37°C for 40 minutes. After confirming that the tissue precipitate is fully digested, 3 ml fetal bovine serum (FBS) (manufactured by JRH) is added, and filtered sequentially with gauze (Type I manufactured by PIP), a 100 µm nylon filter (manufactured by FALCON) and a 40 µm nylon filter (manufactured by FALCON). After centrifuging the resulting filtrate at 1200 rpm and room temperature for 10 minutes to remove the supernatant, DMEM/F-12/1% antibiotics, antimycotics is added to wash the precipitate, and then centrifuged at 1200 rpm and room temperature for 10 minutes. The cell fraction thus obtained is then cultured prior to induction.

Dermal stem cells can be enriched by isolating dermal papilla from scalp tissue. Human scalp tissues (0.5 - 2 cm or less) are rinsed, trimmed to remove excess adipose tissues, and cut into small pieces. These tissue pieces are enzymatically digested in 12.5 mg/ml dispase (Invitrogen, Carlsbad, CA) in DMEM for 24 hours at 4°C. After the enzymatic treatment, the epidermis is peeled off from the dermis; and hair follicles are pulled out from the dermis. Hair follicles are washed with phosphate-buffered saline (PBS); and the epidermis and dermis are removed. A microscope may be used for this procedure. Single dermal papilla derived cells are generated by culturing the explanted papilla on a plastic tissue culture dish in the medium containg DMEM and 10% FCS for 1 week. When single dermal papilla cells are generated, these cells are removed and cultured in FBM supplemented with FGM-2 SingleQuots (Lonza) or cultured in the presence of 20 ng/ml EGF, 40 ng/ml FGF-2, and B27 without serum.

Epidermal stem cells can be also enriched from human scalp tissues (0.5 - 2 cm² or less). Human scalp issues is rinsed, trimmed to remove excess adipose tissues, and cut into small pieces. These tissue pieces are enzymatically digested in 12.5 mg/ml dispase (Invitrogen, Carlsbad, CA) in Dulbecco's modified Eagle's medium (DMEM) for 24 hours at 4°C. After the enzymatic treatment, the epidermis is peeled off from the dermis; and hair follicles are pulled out from the dermis. The bulb and intact outer root sheath (ORS) are dissected under the microscope. After the wash, the follicles are transferred into a plastic dish. Then the bulge region is dissected from the upper follicle using a fine needle. After the wash, the bulge is transferred into a new dish and cultured in medium containing DMEM/F12 and 10 % FBS. After the cells are identified, culture medium is changed to the EpiLife™ Extended-Lifespan Serum-FreeMedium (Sigma).

### 2. Adipose Tissue

Cells derived from adipose tissue for use in the present invention may be isolated by various methods known to a person skilled in the art. For example, such a method is described in U.S. Pat. No. 6,153,432, which is incorporated herein in its entirety. A preferred source of adipose tissue is omental adipose tissue. In humans, adipose cells are typically isolated by fat aspiration.

In one method of isolating cells derived from adipose cells, adipose tissue is treated with 0.01% to 0.5%, e.g., 0.04% to 0.2%, 0.1% collagenase; 0.01% to 0.5%, e.g., 0.04%, or 0.2% trypsin; and/or 0.5 ng/ml to 10 ng/ml dispase, or an effective amount of hyaluronidase or DNase (DNA digesting enzyme), and about 0.01 to about 2.0 mM, e.g., about 0.1 to about 1.0 mM, or 0.53 mM ethylenediaminetetraacetic acid (EDTA) at 25 to 50°C, e.g., 33 to 40°C, or 37°C for 10 minutes to 3 hours, e.g., 30 minutes to 1 hour, or 45 minutes.

Cells are passed through nylon or a cheese cloth mesh filter of 20 microns to 800 microns, more preferably 40 microns to 400 microns, and most preferably 70 microns. Then the cells in the culture medium are subjected to differential centrifugation directly or using Ficoll or Percoll or another particle gradient. The cells are centrifuged at 100 to 3000xg, more preferably 200 to 1500xg, most preferably 500×g for 1 minute to 1 hours, more preferably 2 to 15 minutes and most preferably 5 minutes, at 4 to 50°C, preferably 20 to 40°C and more preferably about 25°C.

The adipose tissue-derived cell fraction thus obtained may be cultured according to the method described herein as crude purified cells containing undifferentiated stem cells, and used for the induction of human pluripotent or multipotent stem cells.

### 3. Blood

About 50 ml to about 500 ml vein blood or cord blood is collected, and a mononuclear cell fraction is obtained by the Ficoll-Hypaque method, as described in, e.g., Kanof et al., (1993), Current Protocols in Immunology (J. E. Coligan, A. M. Kruisbeek, D. H. Margulies, E. M. Shevack, and W. Strober, eds.), ch. 7.1.1.-7.1.5, John Wiley & Sons, New York).

After isolation of the mononuclear cell fraction, approximately 1 x 10⁷ to 1 x 10⁸ human peripheral blood mononuclear cells are suspended in a RPMI 1640 medium containing 10% fetal bovine serum, 100 µg/ml streptomycin and 100 units/ml penicillin, and after washing twice, the cells are recovered. The recovered cells are resuspended in RPMI 1640 medium and then plated in a 100 mm plastic petri dish at a density of about 1 x 10⁷ cells/dish, and incubated in a 37 °C incubator at 8% CO₂. After 10 minutes, cells remaining in suspension are removed and adherent cells are harvested by pipetting. The resulting adherent mononuclear cell fraction is then cultured prior to the induction period as described herein. In some cases, the peripheral blood-derived or cord blood-derived adherent cell fraction thus obtained may be cultured according to the method described herein as crude purified cells containing undifferentiated stem cells, and used for the induction of human pluripotent or multipotent stem cells.

Macrophages in the peripheral blood can be enriched by culturing the mononuclear cell fraction in low-glucose DMEM supplemented with 10% heat-inactivated fetal bovine serum (FBS; JRH Biosciences, Lenexa, KS), 2 mM L-glutamine, 50 U/ml penicillin, and 50 µg/ml streptomycin. In order to expand macrophages, peripheral blood mononuclear cells are spread at a density of 2 x10⁶/ml on plastic plates that have been treated with 10 µg/ml FN (Sigma, St. Louis, MO) overnight at 4°C. The cells are then cultured without any additional growth factors at 37°C and 5% CO2 in a humidified atmosphere. The medium containing floating cells is changed every 3 days. Macrophages with observable fibroblastic features may be used for the induction experiments.

In some cases, a cell fraction from peripheral blood, cord blood, or bone marrow is expanded, as described in U.S. Patent Application Serial No. 11/885,112, and then used in the induction methods described herein.

### D. Generation of iPS Cells

The cells described herein may be induced into iPS using any method known in the art. A common method is to transduce cells with retroviral vectors in order to force expression of Oct3/4, Sox2, Klf4, and c-Myc. Cells may be cultured for a period of time in hES cell medium (or iPS cell medium), after which the induced cells are screened for a number of properties that characterize multipotent and pluripotent stem cells (e.g., morphological, gene expression). Induced cells that meet these screening criteria may then be subcloned and expanded. Cell colonies subcultured from those initially identified on the basis of morphological characteristics may be assayed for any of a number of properties associated with pluripotent stem cells, including, but not limited to, expression of ALP activity, expression of ES cell marker genes, expression of protein markers, hypomethylation of Oct3/4 and Nanog promoters relative to a parental cells, long term self-renewal, normal diploid karyotype, and the ability to form a teratoma comprising ectodermal, mesodermal, and endodermal tissues.

During and following the induction process, the human cells can be cultured in medium such as human ES (or human iPS medium). A typical human iPS medium may be prepared as follows: 390 mL of KO DMEM (Invitrogen); 50 mL Knockout Serum Replacement (Invitrogen); 40 mL Plasmanate (Talecris), 5 mL glutamax (Invitrogen); 5 mL non-essential amino acids (Invitrogen) and 500 uL of bFGF (Invitrogen) for a final conccentration of 20 ng/ml FGF. Mouse iPS cells, or mouse ES cells, can be cultured in any standard mouse ES medium, supplemented with LIF.

The induced cells may be maintained in the presence of a rho, or rho-associated, protein kinase (ROCK) inhibitor to reduce apoptosis. A ROCK inhibitor may be particularly useful when the cells are subjected to a harsh treatment, such as an enzymatic treatment. For example, the addition of Y-27632 (Calbiochem; water soluble) or Fasudil (HA1077: Calbiochem), an inhibitor of Rho associated kinase (Rho associated coiled coil-containing protein kinase) may be used to culture the human pluripotent and multipotent stem cells of the present invention. In some cases the concentration of Y-27632 or Fasudil, is from about 2.5 µM to about 20 µM, e.g., about 2.5 µM, 5 µM, 10 µM, 15 µM, or 20 µM.

The induced cells may be cultured in a maintenance culture medium in a 37 °C, 5% CO₂ incubator (e.g., under an atmospheric oxygen level), with medium changes preferably every day. Examples of maintenance culture media for induced cells include any and all complete ES cell media). The maintenance culture medium may be supplemented with b-FGF or FGF2. In some cases, the maintenance culture medium is supplemented with other factors, e.g., IGF-II, Activin A or other growth factor described herein, see, e.g., Bendall et al., (2007), Nature, 30:448(7157):1015-21. In some embodiments, the induced cells are cultured and observed for about 14 days to about 40 days, e.g., 15, 16, 17, 18, 19, 20, 23, 24, 27, 28, 29, 30, 31, 33, 34, 35, 36, 37, 38 days, or other period from about 14 days to about 40 days, prior to identifying and selecting candidate multipotent or pluripotent stem cell colonies based on morphological characteristics.

Culture of cells may be carried out under low serum culture conditions prior to, during, or following the introduction of induction factors. A "low serum culture condition" refers to the use of a cell culture medium containing a concentration of serum ranging from 0% (v/v) (i.e., serum-free) to about 5% (v/v), e.g., 0% to 2%, 0% to 2.5%, 0% to 3%, 0% to 4%, 0% to 5%, 0.1% to 2%, 0.1% to 5%, 0%, 0.1%, 0.5%, 1%, 1.2%, 1.5%, 2%, 2.5%, 3%, 3.5%, 4%, or 5%. In some embodiments, a low serum concentration is from about 0% (v/v) to about 2% (v/v). In some cases, the serum concentration is about 2%. In other embodiments, cells are cultured under a "high serum condition," i.e., greater than 5% (v/v) serum to about 20% (v/v) serum, e.g., 6%, 7%, 8%, 10%, 12%, 15%, or 20%. Culturing under high serum conditions may occur prior to, during, and/or after the introduction of induction factors. Media with low concentrations of serum may be particularly useful to enrich undifferentiated stem cells. For example, MSCs are often obtained by isolating the non-hematopoietic cells (e.g., interstitial cells) adhering to a plastic culture dish when tissue, e.g., bone marrow, fat, muscle, or skin etc., is cultured in a culture medium containing a high-concentration serum (5% or more). However, even under these culture conditions, a very small number of undifferentiated cells can be maintained, especially if the cells were passaged under certain culture conditions (e.g., low passage number, low-density culturing or low oxygen).

When either low or high serum conditions are used for culturing the cells, one or more growth factors such as fibroblast growth factor (FGF)-2; basic FGF (bFGF); platelet-derived growth factor (PDGF), epidermal growth factor (EGF); insulin-like growth factor (IGF); IGF II; or insulin can be included in the culture medium. Other growth factors that can be used to supplement cell culture media include, but are not limited to one or more: Transforming Growth Factor β-1 (TGF β-1), Activin A, Noggin, Brain-derived Neurotrophic Factor (BDNF), Nerve Growth Factor (NGF), Neurotrophin (NT)-1, NT-2, or NT-3. In some cases, one or more of such factors is used in place of the bFGF or FGF-2 in the MC-ES medium or other cell culture medium.

The concentration of growth factor(s) (e.g., FGF-2, bFGF, PDGF, EGF, IGF, insulin, IGF II, TGF β-1, Activin A, Noggin, BDNF, NGF, NT-1, NT-2, NT-3) in the culture media described herein (e.g., MAPC, FBM, MC-ES, MSCGM, IMDM, mTeSR1^{™}) may be from about 4 ng/ml to about 50 ng/ml, e.g., about 2 ng/ml, 3 ng/ml, 4 ng/ml,5 ng/ml, 6 ng/ml, 7 ng/ml, 8 ng/ml, 10 ng/ml, 12 ng/ml, 14 ng/ml, 15 ng/ml, 17 ng/ml, 20 ng/ml, 25 ng/ml, 30 ng/ml, 35 ng/ml, 40 ng/ml, 45 ng/ml, or 50 ng/ml. The concentration of growth factors may also be from about 4 ng/ml to about 10 ng/ml; from about 4 ng/ml to about 20 ng/ml; from about 10 ng/ml to about 30 ng/ml; from about 5 ng/ml to about 40 ng/ml; or from about 10 ng/ml to about 50 ng/ml. In other cases, higher concentrations of growth factors may be used, e.g., from about 50 ng/ml to about 100 ng/ml; or from about 50 ng/ml to about 75 ng/ml.

The growth factors may be used alone or in combination. For example, FGF-2 may be added alone to the medium; in another example, both PDGF and EGF are added to the culture medium. Often, growth factors appropriate for a particular cell type may be used. For example, dermal cells may be cultured in the presence of about 20 ng/ml EGF and/or about 40 ng/ml FGF-2, while epidermal cells may be cultured in the presence of about 50 ng/ml EGF and/or 5 ug/ml Insulin.

Morphological characteristics for identifying candidate multipotent or pluripotent stem cell colonies include, but are not limited to, a rounder, smaller cell size relative to surrounding cells and a high nucleus-to-cytoplasm ratio. The size of the candidate induced cell may be from about 5 µm to about 10 µm; from about 5 µm to about 15 µm; from about 5 µm to about 30 µm; from about 10 µm to about 30 µm; or from about 20 µm to about 30 µm. A high nucleus-to-cytoplasm ratio may be from about 1.5:1 to about 10:1, e.g., about 1.5:1; about 2:1; about 3:1; about 4:1; about 5:1; about 7:1; about 8:1; about 9.5:1; or about 10:1. In some cases, the induced cell clones display a flattened morphology relative to mouse ES cells. For example, candidate induced cells derived from peripheral blood cells or from cells cultured in feeder-free media may exhibit a flattened morphology compared to surrounding cells. Another morphological characteristic for identifying induced cell clones is the formation of small monolayer colonies within the space between parental cells (e.g., between fibroblasts).

The induced cells can be plated and cultured directly on tissue culture-grade plastic. Alternatively, cells are plated and cultured on a coated substrate, e.g., a substrate coated with fibronectin, gelatin, matrigel™ (BD Bioscience), collagen, or laminin. In some cases, untreated petridishes may be used. Suitable cell culture vessels include, e.g., 35 mm, 60 mm, 100 mm, and 150 mm cell culture dishes, 6-well cell culture plates, and other size-equivalent cell culture vessels. In some cases, the cells are cultured with feeder cells. For example, the cells may be cultured on a layer, or carpet, of MEFs (e.g., irradiated or mitomycin-treated MEFs).

Typically, the induced cells may be plated (or cultured) at a low density, which may be accomplished by splitting the cells from about 1:8 to about 1:3, e.g., about 1:8; about 1:6; about 1:5; about 1:4; or about 1:3. Cells may be plated at a density of from about 10³ cells/cm² to about 10⁴ cells/cm². In some examples, the cells may be plated at a density of from about 1.5 x 10³ cells/cm² to about 10⁴ cells/cm²; from about 2 x 10³ cells/cm² to about 10⁴ cells/cm²; from about 3 x 10³ cells/cm² to about 10⁴ cells/cm²; from about 4 x 10³ cells/cm² to about 10⁴ cells/cm²; or from about 10³ cells/cm² to about 9 x 10³ cells/cm². In some embodiments, the cells may be plated at a density greater than 10⁴ cells/cm², e.g., from about 1.25 x 10⁴ cells/cm² to about 3 x 10⁴ cells/cm².

The iPS cells may be genetically modified in order to express a selectable marker (e.g., antibiotic resistance gene, fluorescent marker such as GFP, mCherry, RFP, YFP) under the control of a cell-type specific promoter. The cells can be genetically modified using the Bacterial Artificial Chromosome (BAC) transgenesis method (described in Example 1), nucleofection, transient transfection, retroviral infection, or any other known method to genetically modify cells. Examples of cell-type specific markers include but are not limited to: HB9 to indicate motor neurons; Chat to indicate motor neurons; ALDh1, GFAP, or S100B to indicate glial cells (e.g., astrocytes; SOX17, FOXA2/A3 to indicate endoderm specification).

### E. Differentiation of iPS

The induced cells may be differentiated into cell-types of various lineages. Examples of differentiated cells include any differentiated cells from ectodermal (e.g., neurons and fibroblasts), mesodermal (e.g., cardiomyocytes), or endodermal (e.g., pancreatic cells) lineages. The differentiated cells may be one or more: pancreatic beta cells, neural stem cells, neurons (e.g., dopaminergic neurons), oligodendrocytes, oligodendrocyte progenitor cells, hepatocytes, hepatic stem cells, astrocytes, myocytes, hematopoietic cells, or cardiomyocytes.

The differentiated cells derived from the induced cells may be terminally differentiated cells, or they may be capable of giving rise to cells of a specific lineage. For example, induced cells can be differentiated into a variety of multipotent cell types, e.g., neural stem cells, cardiac stem cells, or hepatic stem cells. The stem cells may then be further differentiated into new cell types, e.g., neural stem cells may be differentiated into neurons; cardiac stem cells may be differentiated into cardiomyocytes; and hepatic stem cells may be differentiated into hepatocytes.

There are numerous methods of differentiating the induced cells into a more specialized cell type. Methods of differentiating induced cells may be similar to those used to differentiate stem cells, particularly ES cells. In some cases, the differentiation occurs *ex vivo;* in some cases the differentiation occurs *in vivo.*

Motor neurons play a role in amyotrophic lateral sclerosis (ALS) and SMA and are of interest in the present methods. Embryoid body (EB) media can be prepared to maintain EBs formed from iPS cells. EB medium can be prepared with 390 mL KO DMEM (Invitrogen, catalog # 10829-018); 50 mL Knockout Serum Replacement (Invitrogen, catalog# A1099202); 50 mL Plasmanate (Talecris); 5 mL Glutamax (Invitrogen, catalog# 35050079); and 5 mL non-essential amino acids (Invitrogen, cat#11140050))

Embryoid bodies (Ebs) formed from iSCs or iPS cells, can be treated with two small molecules, an agonist of the sonic hedgehog (SHH) signaling pathway and retinoic acid (RA) When the differentiated EBs are allowed to adhere to a laminin-coated surface, neuronal-like outgrowths are observed. These processes then stain positive for a neuronal form of tubulin, β-Tubulin IIIb (TuJI), confirming their neuronal nature. To further characterize the cells after directed differentiation, EBs can be dissociated and plated as a single-cell suspension onto laminin-coated slides. TuJ1-positive neurons that co-express the motor neuron marker HB9 [a motor neuron-specific transcription factor] can be readily identified in cultures. In cultures differentiated from iSCs or iPS cells, greater than 20% of all cells can be expected to express the motor neuron marker HB9. HB9-positive cells may also express ISLET 1/2 (ISL), transcription factors involved in motor neuron development. HB9/ISL positive neurons may also express choline acetyltransferase (ChAT), demonstrating an advanced degree of cholinergic motor neuron maturation. Cells expressing the spinal cord progenitor markers OLIG2 and PAX6 are also observed in such cultures, indicating that patient-specific iSC- (or iPS-cell-) derived motor neurons arise from progenitors similar to those found in the developing spinal cord. In addition, cells expressing the glial markers GFAP and S100 are readily identified. Thus, patient-specific iSCs or iPS cells, like human ES cells, can respond appropriately to developmentally relevant patterning signals, demonstrating the feasibility of producing large numbers of the cells specifically affected by ALS. See, *e.g*., Dimos et al., (2008) Science 321(5893):1218-21, Nagai et al. Nature Neuroscience Vol. 10. No. 5. May 2007. 615-622.; diGiorgio et al. Nature Neuroscience Vol. 10. No. 5. May 2007. 608-614.; P.R. Bar. European Journal of Pharmacology. 405. 2000. 285-295.

In order for EBs to be pre-induced to become motor neurons, they can be resuspended in N2 Base medium supplemented with 1 uM Retinoic Acid (RA) (Sigma, cat. # R2625), and 100 nM Purmorphamine (Cayman Chemical, cat# 483367-10-8). N2 Base medium can be prepared by combining: 489 mL DMEM/F12 + Glutamax (Invitrogen, catalog# 105655 mL N-2 Supplement (Invitrogen, catalog# 17502-048), 4 mL 20% D-Glucose (Sigma, catalog# G8769); and 2 mL of 50 mM Ascorbic Acid (Sigma, catalog# A4403-100mG)).

In order to induce the EBs to become motor neurons, after numerous days in culture, the cells can be resuspended in Motor Neuron Maturation medium, which is prepared by combining: 458 mL DMEM/F12 + Glutamax (Invitrogen, catalog# 10565); 10 mL N-2 Supplement (Invitrogen, catalog# 17502-048); 20 mL B-27 Supplement (Invitrogen, catalog# 17504-044); 8 mL of 20% D-Glucose (Sigma, catalog# G8769); 4 mL of 50 mM Ascorbic Acid (Sigma, catalog# A4403-100mG); and 2ng/mL each of GDNF (R&D, catalog# 212-GD); BDNF (R&D, catalog# 248-BD); and CNTF (R&D, catalog# 257-NT/CF).

Dopaminergic neurons play a central role in Parkinson's Disease and other neurodegenerative diseases and are thus of interest. Dopaminergic neurons may be obtained by specifying iPSCs according to the method of Chambers et al (2009) Nature Biotech 27(3) 275-280. For neural induction, nearly-confluent plates of iPS cells (or ES cells) are incubated with initial differentiation medium (knockout (K/O) serum replacement medium supplemented with 10 uM TGF-b inhibitor (Tocris) and 500 ng/ml of Noggin (R&D)). After five days of differentiation, the TGF-b is withdrawn and increasing amounts of N2 media (25%, 50%, 75%) is added to the K/O serum replacement medium every two days while maintaining 500 ng/ml of Noggin. Dopaminergic patterning is then initiated with the addition of super sonic on days 5-9, followed by the addition of BDNF, ascorbic acid, sonic hedgehog and FGF8. The dopaminergic neurons are then matured with BDNF, ascorbic acid, GDNF, TGFb3 and cAMP, and analyzed for expression of GFP in order to confirm activation of the Pitx3 promoter. Other methods of deriving dopaminergic neurons from ES cells are also described in the art; for example, induced cells may be co-cultured with a PA6 mouse stromal cell line under serum-free conditions, *see, e.g.,* Kawasaki et al., (2000) Neuron, 28(1):31-40. Other methods have also been described, *see, e.g.,* Pomp et al., (2005), Stem Cells 23(7):923-30; U.S. Pat. No. 6,395,546, e.g., Lee et al., (2000), Nature Biotechnol., 18:675-679

Cortical neurons, e.g., cortical pyramidal neurons, which are affected in a number of CNS conditions, e.g., Alzheimer's disease and schizophrenia, may also be derived, e.g., as described in Gaspard et al (2008), Nature, 455(7211):351-357. In one exemplary embodiment, human ES cells or iPS cells are trypsinized, dissociated and plated at a density of 5 x 10³ cells per cm² on gelatin-coated cell culture plastic dishes in ESC medium. After adhesion, medium is changed to defined neural differentiation medium (DNDM). DNDM consists of DMEM/F12 (Invitrogen-Gibco) supplemented with 1 N2 supplement (100 N2 supplement consists of 8.61 µM insulin, 1 mM transferrin, 2 µM progesterone, 10.01 mM putrescine and 3.01 µM selenite; Invitrogen-Gibco), 2 mM glutamine, 1X MEM-nonessential amino acids, 1 mM sodium pyruvate, 0.5 mg/ml bovine serum albumin (BSA) fraction V (all from Invitrogen-Gibco), and 110 µM -mercaptoethanol (Sigma). Cyclopamine (Calbiochem) is added from day 2 to day 10 in the differentiation medium at a final concentration of 1 µM. After 12 to 18 days of differentiation, cells are trypsinized, dissociated, and plated on polylysine/laminin (Becton-Dickinson) coated glass coverslips, and cultured for 30 to 90 days (e.g., 30, 35, 40, 45, 50, 55, 60, 70, or 75 day) to permit synaptogenesis and synapse maturation in N2B27 medium to allow synapse formation and maturation. N2B27 medium consists of a 1:1 mixture of DMEM/F12 supplemented with 1 N2, 2 mM glutamine, 0.5 mg/ml BSA fraction V and 110 µM β-mercatoethanol with Neurobasal supplemented with B27 (without vitamin A; Invitrogen-Gibco) and 2 mM glutamine.

Oligodendrocytes may also be generated from the induced cells. Differentiation of the induced cells into oligodendrocytes may be accomplished by known methods for differentiating ES cells or neural stem cells into oligodendrocytes. For example, oligodendrocytes may be generated by co-culturing induced cells or neural stem cells with stromal cells, e.g., Hermann et al. (2004), J Cell Sci. 117(Pt 19):4411-22. In another example, oligodendrocytes may be generated by culturing the induced cells or neural stem cells in the presence of a fusion protein, in which the Interleukin (IL)-6 receptor, or derivative, is linked to the IL-6 cyotkine, or derivative thereof. Oligodendrocytes can also be generated from the induced cells by other methods known in the art, see, e.g. Kang et al., (2007) Stem Cells 25, 419-424.

Astrocytes may also be produced from the induced cells. For example, astrocytes may be derived by using the same conditions used to generate motor neurons, except that the progenitors are cultured in maturation medium for a longer period of time (e.g., 30 to 45 days). Astrocytes may be generated by culturing induced cells or neural stem cells in the presence of neurogenic medium with bFGF and EGF, *see e.g*., Brustle et al., (1999), Science, 285:754-756; Benveniste et al., (2005), J. Neurosurg. 115-123.

Any known method of generating neural stem cells from ES cells may be used to generate neural stem cells from induced cells, *See, e.g.,* Reubinoff et al., (2001), Nat, Biotechnol., 19(12):1134-40. For example, neural stem cells may be generated by culturing the induced cells as floating aggregates in the presence of noggin, or other bone morphogenetic protein antagonist, see e.g., Itsykson et al., (2005), Mol, Cell Neurosci., 30(1):24-36. In another example, neural stem cells may be generated by culturing the induced cells in suspension to form aggregates in the presence of growth factors, e.g., FGF-2, Zhang et al., (2001), Nat.Biotech., (19):1129-1133. In some cases, the aggregates are cultured in serum-free medium containing FGF-2. In another example, the induced cells are co-cultured with a mouse stromal cell line, e.g., PA6 in the presence of serum-free medium comprising FGF-2. In yet another example, the induced cells are directly transferred to serum-free medium containing FGF-2 to directly induce differentiation.

Neural stem cells derived from the induced cells may be differentiated into neurons, oligodendrocytes, or astrocytes. Often, the conditions used to generate neural stem cells can also be used to generate neurons, oligodendrocytes, or astrocytes.

Induced cells may be differentiated into pancreatic beta cells by methods known in the art, e.g., Lumelsky et al., (2001) Science, 292:1389-1394; Assady et al., (2001), Diabetes, 50:1691-1697; D'Amour et al., (2006), Nat. Biotechnol., 24:1392-1401; D'Amour et al., (2005), Nat. Biotechnol. 23:1534-1541. To derive pancreatic beta cells, cells can be first induced to become definitive endoderm by culturing in the presence of high concentrations of Activin A and low serum as described in D'Amour et al. (2005) Nature Biotechnol 23:1534-41, or high concentrations of Activin and low doses of Wnt3A. Differentiation can be monitored using cell surface markers CXCR4 and CKIT and intracellular markers SOX17, FOXA2, FOXA3 and the absence of SOX7. The definitive endoderm is then specified to the pancreatic lineage using retinoic acid, Cyclopamine, Noggin, Exendin 4, DAPT and FGF10. Pancreatic beta cell differentiation is determined by monitoring PDX1 and NGN3 expression and insulin release (c-peptide) after glucose stimulation.

Methods of specifying pancreatic beta cells may comprise culturing the induced cells in serum-free medium supplemented with Activin A, followed by culturing in the presence of serum-free medium supplemented with all-trans retinoic acid, followed by culturing in the presence of serum-free medium supplemented with bFGF and nicotinamide, e.g., Jiang et al., (2007),Cell Res., 4:333-444. In other examples, the method comprises culturing the induced cells in the presence of serum-free medium, activin A, and Wnt protein from about 0.5 to about 6 days, e.g., about 0.5, 1, 2, 3, 4, 5, 6, days; followed by culturing in the presence of from about 0.1% to about 2%, e.g., 0.2%, FBS and activin A from about 1 to about 4 days, e.g., about 1, 2, 3, or 4 days; followed by culturing in the presence of 2% FBS, FGF-10, and KAAD-cyclopamine (keto-N-aminoethylaminocaproyl dihydro cinnamoylcyclopamine) and retinoic acid from about 1 to about 5 days, e.g., 1, 2, 3, 4, or 5 days; followed by culturing with 1% B27, gamma secretase inhibitor and extendin-4 from about 1 to about 4 days, e.g., 1, 2, 3, or 4 days; and finally culturing in the presence of 1% B27, extendin-4, IGF-1, and HGF for from about 1 to about 4 days, e.g., 1, 2, 3, or 4 days.

In order to specify adipocytes, iPS cells may be subjected to the conditions provided in Taura et al. (2009) FEBS Letters 583: 1029-33 are followed. Briefly, EBs may be formed from the iPS cells, in the presence of retinoic acid and / or BMP4 and Activin A. Cells may be later transferred to Poly L-ornithine/fibronectin plates, followed by differentiation in the presence of DMEM-F12, 10% KSR or fetal calf serum or defined growth factors (BMP4, FGF, VEGF) and an adipogenic cocktail (e.g., IBMX, dexamethasone, insulin, indomethacin and pioglitazone). Alternatively, for the methods and compositions described herein, adipocytes may be derived from subjects (e.g., human) following liposuction or other fat-removal procedure.

Hepatic cells or hepatic stem cells may be differentiated from the induced cells. For example, culturing the induced cells in the presence of sodium butyrate may generate hepatocytes, *see e.g.,* Rambhatla et al., (2003), Cell Transplant, 12:1-11. In another example, hepatocytes may be produced by culturing the induced cells in serum-free medium in the presence of Activin A, followed by culturing the cells in fibroblast growth factor-4 and bone morphogenetic protein-2, e.g., Cai et al., (2007), Hepatology, 45(5):1229-39. In an exemplary embodiment, the induced cells are differentiated into hepatic cells or hepatic stem cells by culturing the induced cells in the presence of Activin A from about 2 to about 6 days, e.g., about 2, about 3, about 4, about 5, or about 6 days, and then culturing the induced cells in the presence of hepatocyte growth factor (HGF) for from about 5 days to about 10 days, e.g., about 5, about 6, about 7, about 8, about 9, or about 10 days.

The induced cells may also be differentiated into cardiac muscle cells. Inhibition of bone morphogenetic protein (BMP) signaling may result in the generation of cardiac muscle cells (or cardiomyocytes), see, e.g., Yuasa et al., (2005), Nat. Biotechnol., 23(5):607-11. Thus, in an exemplary embodiment, the induced cells are cultured in the presence of noggin for from about two to about six days, e.g., about 2, about 3, about 4, about 5, or about 6 days, prior to allowing formation of an embryoid body, and culturing the embryoid body for from about 1 week to about 4 weeks, e.g., about 1, about 2, about 3, or about 4 weeks.

In other examples, cardiomyocytes may be generated by culturing the induced cells in the presence of leukemia inhibitory factor (LIF), or by subjecting them to other methods known in the art to generate cardiomyocytes from ES cells, e.g., Bader et al., (2000), Circ. Res., 86:787-794, Kehat et al., (2001), J Clin. Invest., 108:407-414; Mummery et al., (2003), Circulation, 107:2733-2740.

Examples of methods to generate other cell-types from induced cells include: (1) culturing induced cells in the presence of retinoic acid, leukemia inhibitory factor (LIF), thyroid hormone (T3), and insulin in order to generate adipocytes, e.g., Dani et al., (1997), J. Cell Sci., 110:1279-1285; (2) culturing induced cells in the presence of BMP-2 or BMP-4 to generate chondrocytes, e.g., Kramer et al., (2000), Mech. Dev., 92:193-205; (3) culturing the induced cells under conditions to generate smooth muscle, e.g., Yamashita et al., (2000), Nature, 408:92-96; (4) culturing the induced cells in the presence of beta-1 integrin to generate keratinocytes, e.g., Bagutti et al., (1996), Dev. Biol., 179:184-196; (5) culturing the induced cells in the presence of Interleukin-3(IL-3) and macrophage colony stimulating factor to generate macrophages, e.g., Lieschke and Dunn (1995), Exp. Hemat., 23:328-334; (6) culturing the induced cells in the presence of IL-3 and stem cell factor to generate mast cells, e.g., Tsai et al., (2000), Proc. Natl. Acad. Sci. USA, 97:9186-9190; (7) culturing the induced cells in the presence of dexamethasone and stromal cell layer, steel factor to generate melanocytes, e.g., Yamane et al., (1999), Dev. Dyn., 216:450-458; (8) co-culturing the induced cells with fetal mouse osteoblasts in the presence of dexamethasone, retinoic acid, ascorbic acid, beta-glycerophosphate to generate osteoblasts, e.g., Buttery et al., (2001), Tissue Eng., 7:89-99; (9) culturing the induced cells in the presence of osteogenic factors to generate osteoblasts, e.g., Sottile et al., (2003), Cloning Stem Cells, 5:149-155; (10) overexpressing insulin-like growth factor-2 in the induced cells and culturing the cells in the presence of dimethyl sulfoxide to generate skeletal muscle cells, e.g., Prelle et al., (2000), Biochem. Biophys. Res. Commun., 277:631-638; (11) subjecting the induced cells to conditions for generating white blood cells; or (12) culturing the induced cells in the presence of BMP4 and one or more: SCF, FLT3, IL-3, IL-6, and GCSF to generate hematopoietic progenitor cells, e.g., Chadwick et al., (2003), Blood, 102:906-915.

In some cases, sub-populations of differentiated cells may be purified or isolated. In some cases, one Or more monoclonal antibodies specific to the desired cell type are incubated with the cell population and those bound cells are isolated. In other cases, the desired subpopulation of cells expresses a reporter gene that is under the control of a cell type specific promoter.

In a specific embodiment, the hygromycin B phosphotransferase-EGFP fusion protein is expressed in a cell type specific manner. The method of purifying comprises sorting the cells to select green fluorescent cells and reiterating the sorting as necessary, in order to obtain a population of cells enriched for cells expressing the construct (e.g., hygromycin B phosphotransferase-EGFP) in a cell-type-dependent manner. Selection of desired sub-populations of cells may also be accomplished by negative selection of proliferating cells with the herpes simplex virus thymidine kinase/ganciclovir (HSVtk/GCV) suicide gene system or by positive selection of cells expressing a bicistronic reporter, e.g., Anderson *et al.* (2007) *Mol Ther.* (11):2027-2036.

### IV. Co-Cultures for Drug Discovery

The co-cultures described herein can serve as the basis of screening assays designed to identify compounds capable of correcting a phenotype described herein. Identified compounds may also be capable of intercepting the action of a soluble factor, where a phenotype is due to a cell-non-autonomous process.

The genotypes of iSC and iPS cell lines and those of the corresponding subject are identical. Thus, genotype-phenotype correlations, uncovered in one may be informative for the other, and *vice versa.* In addition, differentiated cells (e.g., neurons) derived *ex vivo* from an iSC line may exhibit a complete set of cellular phenotypes (referred to herein as a "phenome") that are very similar, if not identical, to those of differentiated cells *in vivo* in the corresponding subject. This point is particularly relevant for developing therapeutics targeted to cells that cannot be routinely obtained from patients such as motor neurons. For example, in the case of a patient suffering from a neurodegenerative disease such as Parkinson's Disease, dopaminergic neurons, which are typically affected by this condition, can be obtained via a minimally invasive procedure by differentiating an iSC line or iPS cell line from, for example, skin or blood cells of the subject, and can then be screened in multiple assays. Thus, iSC lines or iPS cell lines provide a renewable source of differentiated cells (e.g., inaccessible differentiated cells) in which pathological cellular phenotypes that are specific to a disease, cell type, and individual may be examined and screened against test compounds. iSC lines or iPS cell lines are also useful for predicting the efficacy and/or adverse side effects of a candidate drug compound in specific individuals or groups of individuals. For example, test compounds can be tested for toxicity in somatic cells differentiated from a genetically diverse panel of iSCs or iPS cells. Toxicity testing in iSC- or iPS-cell-derived cells can reveal both the overall likelihood of toxicity of a test compound in a target patient population, and the likelihood of toxicity in specific patients within that population.

In effect, iSC lines or iPS cells, and cells derived from them, can serve as "cellular avatars," that reveal cellular phenotypes that are disease, cell-type, and subject-specific to the extent the phenotypes are determined by the genome. Collectively, panels of induced stem cell lines will represent a wide range of genotype/phenotype combinations in a patient population. Thus, they are useful for developing therapeutics that are effective and safe across a wide range of the relevant target population, or for determining which individuals can be treated effectively and safely with a given therapeutic agent.

### A. Screening Test Agents for Lead Compounds that Modulate a Cellular Phenotype

ALS is characterized by the death of motor neurons in the cortex, the brain stem, and the spinal cord. ALS is familial in about 10% of the cases and is sporadic in the rest. The course of the disease is indistinguishable between familial and sporadic ALS. The disease manifests itself late in life at an average age of 56 years, and once diagnosed, leads to complete paralysis and death within 2-5 years.

In a subset of the familial patients, mutations have been found in a gene coding for copperzinc superoxide dismutase 1 (SOD1). SOD1 is a cytosolic enzyme involved in detoxification of free radicals. Further, overproduction of pathogenic human SOD1 protein encoding alleles in motor neurons leads to late-onset, progressive neurodegenerative disease. Studies have led to the identification of intrinsic pathogenic characteristics of ALS motor neurons, including the formation of protein aggregates, cytoskeletal abnormalities, proteosome dysfunction and increased sensitivity to cell death signals. Additionally, overproduction of pathogenic human SOD1 protein encoding alleles in other cells often found associated with motor neurons *in vivo,* such as but not limited to glial cells (e.g. Schwann cells, astrocytes etc.) may result in non-cell autonomous motor neuron pathologies. Examples of a pathogenic SOD1 protein encoding allele include but are not limited to SOD1G93A, SOD1G85R, and SOD1G37R.

In some embodiments of the present invention, methods and compositions are provided for the identification, characterization and optimization of lead compounds that exhibit neuroprotective effects in an ALS disease model system. In one aspect of the present invention, wild-type motor neurons are cultured in the presence of SOD1G93A expressing glial cells (e.g. astrocytes) or glial cells (e.g., astrocytes) from a subject with sporadic ALS. In some cases the motor neurons and/or astrocytes are derived from iSCs. In others, they are derived from iPSCs. In some cases, in the absence of any neuroprotective factors, a significantly greater number of motor neurons die after about 14 days in culture as compared to motor neuron death during co-culture with wild-type glial cells. In some cases, cells are co-cultured for about two weeks and there is about a 50% increase in motor neuron death when co-cultured in the presence of SOD1G93A glial cells without any neuroprotective agents. In other cases, motor neurons may be cultured for about 1 day to about 5 weeks including about 1 day, 2 days, 4 days, 6 days, one week, 10 days, 12 days, 15 days, 20 days, 25 days, 30 days or more. In some cases, the motor neurons themselves express an SOD1 disease allele. In other cases, the motor neurons (wild-type, or SOD1 disease allele expressing) are cultured alone, i.e. without supporting glial cells. In some cases, the presence of a neuroprotective compound in the media of the cultured motor neurons leads to an increase in cell viability or a decrease in cell death.

Alternatively, in some embodiments, wild-type motor neurons may be cultured in the presence of SOD1 disease allele expressing glial cell (e.g. astrocyte) conditioned medium. In some cases, motor neurons are cultured for about 7 days, or from about 1 day to about 30 days, in wild-type or SOD1 disease allele expressing glial cell conditioned media. Conditioned media from SOD1 disease allele expressing glial cells in this case may lead to approximately 10%-75% increased motor neuron death in comparison to culture of motor neurons in wild-type glial cell conditioned media. In some cases, the presence of a neuroprotective compound in the media of the cultured motor neurons leads to an increase in cell viability or a decrease in cell death.

Other motor neurons, or glia cells (e.g., astrocytes) that can be used in the disclosed methods and compositions are neurons or glia derived from a subject with one or more SNPs associated with ALS. For example, rs1260832, located at 19p13.3 and maps to an intron within the UNC13A gene is associated with ALS. Also, rs2814707 and rs3849942, both located at chromosome 9p21.2, are also associated with ALS.

In some embodiments of the present invention, a gene encoding an easily detectable marker gene may be incorporated into glial cells, motor neurons or both. Examples of such marker genes include but are not limited to fluorescent proteins such as GFP, YFP, RFP, Timer GFP, beta-lactamase, etc.; beta galactosidase, beta glucuronidase, luciferase etc.. Said marker genes may in some cases facilitate enumeration of cells, and allow cells derived from different subjects to be distinguished when co-cultured. The use of different marker genes for different cell types, or a marker gene for one cell type and no marker gene for another may be particularly important for accurately counting cells in a co-culture system. In some cases, cell counting may be performed by microscopic examination of cells with or without cytlogical or immunohistochemical staining. In other cases, cells may be counted by flow cytometry. In some cases, flow cytometry analysis may be facilitated by labeling cells for markers specific for motor neurons and or glial cells.

Candidate neuroprotective lead compounds may be added to the culture media of cells of the present invention and assayed for desireable properties. Compounds exhibiting desireable properties such as compounds that increase the number of viable wild-type or SOD1 disease allele expressing motor neurons in a culture relative to the number of viable motor neurons in the absence of the candidate neuroprotective lead compound may be selected for further characterization and optimization. Alternatively, compounds may be selected for their ability to increase the viability of motor neurons (wild-type or SOD1 disease allele expressing) in a co-culture with SOD1 disease allele expressing glial cells (e.g. astrocytes), or in media conditioned by SOD1 disease allele expressing glial cells.

Neuroprotective compounds may include but are not limited to compounds related to the membrane permeant pentapeptide VPMLK (V5), which inhibits the death agonist Bax, peptide derivatives or mimetics of said pentapeptide, other inhibitors of Bax, inhibitors of the Bax induced cell death pathway, general inhibitors of apoptosis, small organic molecules, nucleic acids, nucleic acid analogues, proteins, carbohydrates, antibodies, natural products, or any molecule that increases the viability of motor neurons as shown by the methods of the present invention.

In addition to neuroprotective compounds, the high-throughput screens may also be used to identify compounds capable of correcting any disease-associated phenotype, e.g., survival, apoptosis, necrosis, axonal degeneration, axonal guidance, axonal morphology, dendritic morphology, receptor density, synaptogenesis, neurogenesis, synapse density, synaptic transmission, synaptic signaling, receptor trafficking, protein trafficking, protein aggregation, proteasome activity, receptor expression, oxidative stress (ROS), FGFR-signaling, FGF signaling, mitochondrial activity, mitochondrial distribution, mitochondrial morphology, insulin-mediated glucose uptake, and mRNA expression profile

Characterization and optimization of candidate lead compounds selected for their ability to modulate a cellular phenotype (e.g., neuroprotective effect) include but is not limited to further screening, generation of derivatives and analogues, analysis of Absorption Distribution, Metabolism, and Excretion (ADME) characteristics, safety trials, and efficacy trials. In particular, optimization efforts may involve developing compounds which are designed to cross the blood brain barrier.

The iSC lines, iPS cell lines and panels of iSC- and iPS-cell- derived cells described herein are useful in a number of methods relating to drug discovery and development. Typically, a drug candidate compound is evaluated in a biochemical assay (e.g., a receptor binding assay) that evaluates only a single or very few sequence variants of the drug target expressed in a patient population. Thus, such assays provide little information as to how effective the drug candidate compound is likely to be in patients that express a drug target allele that differs from the particular drug target allele that was originally screened. Along the same lines, drug candidate compounds often undergo functional cellular screens in one or few cell lines engineered to express a specific allele of the drug target, again ignoring the genetic diversity of a human patient population not only with respect to the drug target itself, but also to that of the various downstream signal transduction proteins that play a role in the response endpoint of cells to a drug. Likewise, adverse effects of candidate drug compounds (e.g., liver toxicity) are generally evaluated in inbred animal models, which are likely to be uninformative for a variable fraction of a human patient population. In contrast, drug screening in panels of genetically diverse iSC lines or iPS cell lines, as described herein, addresses the lack of genetic diversity in the prevailing drug screening models.

The panels of genetically diverse iSC lines described herein (e.g., human iSC lines, iPS cell lines) or cells differentiated from panels of genetically diverse iSC lines or iPS cell lines, as described herein, may be used to identify test compounds that act on a drug target of interest. In some embodiments, the panels of iSCs cell lines or iPS cell lines include a sufficient number of iSC lines or iPS cell lines such that at least two, e.g., at least 3, 5, 10, 20, 50, 100, or 200 polymorphic alleles of a drug target (e.g., a GPCR, ion channel, or kinase) are represented in the panel. In some embodiments, panels of iSC lines or iPS cell lines are derived from subjects diagnosed as suffering from a health condition or identified as having a predisposition to the health condition. In other embodiments, the iSC line panels or iPS cell line panels comprise iSC lines or iPS cell lines, each of which has at least one polymorphic allele associated with a health condition or a predisposition to the health condition.

Drug targets for many health conditions are known. Such drug targets may include, but are not limited to, receptors, GPCRs, growth factor receptors, neurotransmitter receptors, ion channels, enzymes, protein kinases, proteases, cytoskeletal proteins, and transcription factors. Test compounds can be assayed for their effect on a drug target by a number of assays known in the art. Such assays include cell-based assays including, but not limited to, assays for determining second messenger levels, e.g., intracellular calcium, cAMP, cGMP, arachidonic acid, and inositol phosphates; channel currents; apoptosis; proliferation; morphological changes; changes in adhesion, insulin-mediated glucose uptake. Examples of cell-based assays include, but are not limited to those described in, U.S. Patent Nos. 7,319,009, 7,288,368, and 7,238,213, Cell based assays may also include determining the cellular localization of one or more proteins (e.g., protein kinases, receptors, and transcription factors) in cells in the presence or absence of a test compound. Test compounds may also be screened for their ability to alter a gene expression profile by any gene expression profiling method known in the art. In some cases, the cells to be screened may be genetically modified to express one or more reporter proteins that can indicate activation of a signaling pathway. For example protein-protein interactions between fusion proteins introduced into cells may be detected by a number of methods known in the art, e.g., by fluorescence resonance energy transfer (FRET) or enzyme fragment complementation.

In some cases, the drug target for a health condition of interest is not known. In such cases, it may be advantageous to monitor a phenotypic output associated with said health condition (e.g., abnormal cell morphology, gene expression, viability, death, or signaling). For example, in the case of neurodegenerative disease, neural cell death may be associated with a neurodegenerative health condition. In such cases, cell viability may be monitored rather than the output of a particular protein, enzyme, or pathway. Methods for determining cell viability include but are not limited to: the MTS assay in which 3-(4,5-dimethylthiazol-2-yl)-5-(3-carboxymethoxyphenyl)-2-(4-sulfophenyl)-2H-tetrazolium, inner salt is reduced to formazan by intracellular dehydrogenase activity in metabolically active cells; MTT assay; propidium iodide staining; tunel assay; ethidium homodimer exclusion assay; and europium or chromium release assay, and annexin V staining.

In some cases, a disease allele known to cause or contribute to a health condition is not the drug target. For example, in the case of ALS, familial disease alleles include alleles of SOD 1. However, drug targets for tretment of ALS may include but are not limited to members of the Bax induced death pathway, proteins and enzymes related to glutamate production, and mitochondrial electron transport proteins. In such cases it may still be advantageous to screen differentiated cells from large numbers of genetically distinct iSC cell lines to ascertain safety, and efficacy, even though there may be few polymorphisms between these cells at the SOD1 locus.

Such assays may include contacting a test population of iSC- or iPS-cell-derived cells from one or more iSC or iPS donors with a test compound (or test agent) and contacting with a negative control compound a negative control population of iSC-derived cells from the same one or more iSC donors. The assayed cellular phenotype associated with the health condition of interest in the test and negative control populations can then be compared to a normal cellular phenotype. Where the assayed cellular phenotype in the test population is determined as being closer to a normal cellular phenotype than that exhibited by the negative control population, the drug candidate compound is identified as normalizing the phenotype. A normal cellular phenotype with respect to a particular health condition or a predisposition for a health condition may be established in iSC-derived cells from iSC donors that do not suffer from the health condition or a predisposition for the health condition.

A test agent includes any candidate compound. Assays of drug candidate compounds in an iSC line, a panel of iSC lines, or a panel of differentiated cells from iSC lines can include determining a dose-response. In some embodiments, the dose response of an iSC line or that of one or more types of cells differentiated from the iSC line provides an indication that of the likely efficacy of the compound in the corresponding iSC donor. In some embodiments, the fraction of iSC lines in a panel of cells that exhibit an acceptable dose-response to a test compound indicates an expected probability of an acceptable dose-response relationship in the target patient population of interest. In some cases, cell-based assays of drug candidate include a comparison of responses obtained in a panel of iSC lines or iSC-derived cells to one or more reference iSC lines or iSC derived cells that serve as a positive or negative control for the effect of a drug candidate compound. The reference cells or cell lines may be from a healthy iSC donor, from an iSC donor diagnosed as suffering from a health condition, or an iSC donor carrying a polymorphic allele associated with a health condition. In other embodiments, assays of drug candidate compounds in iSC lines or cells derived from iSC cell lines can include determining effective concentrations, maximum tolerated dose and minimum effective concentration. Additional methods and assays are disclosed in US application WSGR Docket Number 36588-707.101; filed 6/13/08; First Inventor Kazuhiro Sakurada, hereby incorporated by reference.

In some cases, the drug screening may be conducted on cells differentiated from induced cells. Examples of such differentiated cells are described herein (e.g., hepatic cells, neural stem cells, neurons, pancreatic beta cells, cardiomyocytes, hepatic stem cells, oligodendrocytes). The drugs may be targeted to treat a specific disease or condition, e.g., a disease or condition described herein. For example, the induced cells may be differentiated into dopaminergic neurons, which are used to screen drugs for Parkinson's disease. In other cases, neurons or neural stem cells differentiated from induced cells may be used to screen drugs for treating Alzheimer's disease, ALS, Parkinson's disease, Wilson's disease, Huntington's disease, multiple sclerosis, or other neurological disorders. In other cases, the induced cells may be transplanted directly into an immunocompromised animal, e.g., SCID mouse, which is then used to establish in vitro or in vivo assay systems that mimic physiologic conditions in humans or other animals. The in vitro or in vivo assay systems may be used to screen for drugs, e.g., drugs for Parkinson's disease, or as a means to identify biological mechanisms.

Test compounds (or test agents) identified as lead compounds, may be tested on a panel of iSC-derived cells in a manner analogous to a clinical trial. In some cases, the efficacy of the lead compound versus a negative control compound, e.g., a placebo compound is determined in a panel of iSC-derived cells from patients suffering from the same health condition. Preferably, such a panel of iSC-derived cells is from subjects that are genetically diverse. For example, such patients may be carry at least one polymorphic allele that is unique among the iSC-derived cells to be included in the panel, e.g., 5 to 10, 20 to 50, 50 to 200, 200 to 500, 500 to 1000, 1000 to 5000, 5000 to 20000, or 20000 to 50000 polymorphic alleles that are unique within the panel of iSC lines. A number of methods for quantifying the genetic diversity of a population are known in the art, e.g., the analysis of molecular variance (AMOVA) and generalized analysis of molecular variance (GAMOVA). See, e.g., Excoffier et al (1992), Genetics, 131: 479-491; Nievergelt et al (2008), PLOS Genetics, 3(4):e51. Various clinical experimental designs known in the art may be used for comparing the effect of a lead compound versus a negative control compound. See, e.g., Chow et al (2004) "Design and Analysis of Clinical Trials: Concepts and Methodologies," John Wiley & Sons, Inc., Hoboken, NJ.

The efficacy of the lead compound in iSC-derived cells may be determined based on any cellular response endpoint, e.g., a response obtained in any of the cell-based assays or gene expression profiling assays mentioned herein.

In some cases, potential adverse effects of a lead compound are tested on a panel of iSC-derived cells. The iSC-derived cells may include any cell type such as hepatocytes, cardiomyocytes, neurons,

Drug candidate compounds (or "test agents") may be individual small molecules of choice (e.g., a lead compound from a previous drug screen) or in some cases, the drug candidate compounds to be screened come from a combinatorial library, i.e., a collection of diverse chemical compounds generated by either chemical synthesis or biological synthesis by combining a number of chemical "building blocks." For example, a linear combinatorial chemical library such as a polypeptide library is formed by combining a set of chemical building blocks called amino acids in every possible way for a given compound length (i.e., the number of amino acids in a polypeptide compound). Millions of chemical compounds can be synthesized through such combinatorial mixing of chemical building blocks. Indeed, theoretically, the systematic, combinatorial mixing of 100 interchangeable chemical building blocks results in the synthesis of 100 million tetrameric compounds or 10 billion pentameric compounds. See, e.g., Gallop et al. (1994), J. Med. Chem 37(9), 1233. Preparation and screening of combinatorial chemical libraries are well known in the art. Combinatorial chemical libraries include, but are not limited to: diversomers such as hydantoins, benzodiazepines, and dipeptides, as described in, e.g., Hobbs et al. (1993), Proc. Natl. Acad. Sci. U.S.A. 90, 6909; analogous organic syntheses of small compound libraries, as described in Chen et al. (1994), J. Amer. Chem. Soc., 116: 2661; Oligocarbamates, as described in Cho, et al. (1993), Science 261, 1303; peptidyl phosphonates, as described in Campbell et al. (1994), J. Org. Chem., 59: 658; and small organic molecule libraries containing, e.g., thiazolidinones and metathiazanones (U.S. Pat. No. 5,549,974), pyrrolidines (U.S. Pat. Nos. 5,525,735 and 5,519,134), benzodiazepines (U.S. Pat. No. 5,288,514).

Numerous combinatorial libraries are commercially available from, e.g., ComGenex (Princeton, NJ); Asinex (Moscow, Russia); Tripos, Inc. (St. Louis, MO); ChemStar, Ltd. (Moscow, Russia); 3D Pharmaceuticals (Exton, PA); and Martek Biosciences (Columbia, MD).

In some embodiments of the present invention, a screen may be performed in a high throughput fashion. Such high throughput screening methodologies include but are not limited to screening of cells in multi-well plates such as 24, 48, 96, 384, or 1536 well plates. Other methodologies for high throughput screening include embedding cells in a porous matrix such as a gel such as described in US patent application no. 08/990,168.

Provided herein is a method of co-culturing differentiated cells derived from iSCs that are two or more differentiated cell types from a first human subject and a second human subject in the presence or absence of a test agent; and indicating that the test agent is a cytoprotective agent if survival of the co-cultured differentiated cells from the first subject is greater in the presence of the test agent or indicating that the test agent is not a cytoprotective agent if the survival in the presence of the test agent is the same or less than in the absence of the test agent, wherein the first human subject is normal and the second human subject is identified as suffering from or prediposed to a disorder. In some embodiments, the methods comprise culturing a first differentiated cell type derived from iSCs in the presence of conditioned medium from a second differentiated cell type derived from iSCs, where it is indicative that the test agent is a neuroprotective agent if survival of the first differentiated cell type is greater in the presence of the test agent or indicating that the test agent is not a neuroprotective agent if the survival in the presence of the test agent is the same or less than in the absence of the test agent, wherein the first differentiated cell type is normal and the second differentiated cell type is identified as suffering from or predisposed to a neurodegenerative condition.

Coculture assays are known in the art. In some embodiments, the iSC-derived differentiated cells from the first and second subject are neural cells. In some embodiments, the neural cells are motor neurons and astrocytes. The coculture of motor neurons and astrocytes to characterize non-cell autonomous effects of astrocyte-conditioned media are known in the art. See, *e.g.,* Nagai et al. Nature Neuroscience Vol. 10. No. 5. May 2007. 615-622.; diGiorgio et al. Nature Neuroscience Vol. 10. No. 5. May 2007. 608-614. In some embodiments of the presently claimed methods, iSC-derived neuronal and astrocytic cells can be cocultured in the presence of test agents in order to assess their effects on non-cell autonomous toxicity.

### EXAMPLES

### Example 1 (prophetic example) Co-cultures of motor neurons to model SMA

This example illustrates a co-culture of motor neurons (MN) derived from human iPS cells from healthy human subjects and human subjects having, or predisposed to, SMA ("SMA subjects") in order to model SMA disease. This example makes use of a competition assay, in which MNs are cultured with healthy muscle tissue and allowed to undergo a synaptic competition, wherein the weaker MNs retract their axons, and the stronger MNs with the highest level of neuronal activity (and transmitter release) remain to innervate the muscle. Once the model is established, it can be used to screen for compounds that improve the activity of the SMA MNs.

The human iPS cells may either be normal or genetically modified to express a fluorescent marker under the control of the promoter of a MN marker (e.g., HB9). In this example, healthy iPS cells are labeled so that they express GFP (green color) when differentiated into MNs; and the iPS cells from SMA subjects are labeled so that they express cherry (red color) when differentiated into MNs.

**Generation of iPS cells**: Induction of iPS cells is initiated by transduction of fibroblast cultures with four MoMLV VSV-G-pseudotyped viruses for expression of human OCT4, SOX2, KLF4, and c-MYC, each at an MOI of about 10. Five days after viral transduction, fibroblasts are switched from human fibroblast medium into human ES cell supportive medium and monitored daily for the appearance of putative iPS cell colonies based on morphological criteria.

Initial putative iPS cell colonies are picked after approximately three weeks and propagated clonally (and sometimes trypsinized and replated in the presence of the ROCK inhibitor Y-27632 (10 µM) Calbiochem)) to derive iPS cell lines. The iPS cells are analyzed for pluripotency associated markers such as Nanog, Oct4, SSEA3, SSEA4, TRA1-60, an/or TRA1-81 by immunocytochemistry. Q-PCR analysis is used to show that these iPS cell lines express endogenous Oct 4, Sox2, and Klf4, but not the exogenous Oct4, Sox2, and Klf4 introduced by viral transduction. In addition, Q-PCR analysis is also used to determine expression ofNanog, SSEA 3, SSEA-4, TRA1-60, TRA1-81, DNMT3B, FOXD3, LIN28, ZNF206, LEFT2, TDGF1, and TDGF2 in all of the iPSC lines.

**Introduction of Selectable Markers**: Bacterial artificial chromosome (BAC) transgenesis is used to generate cell-type specific reporter lines that read out the motor neuron fate. BACs are engineered to express either GFP or mCherry under the control of the HB9 promoter and modified to express a neomycin resistance gene. A single-cell suspension of iPS cells from each subject is prepared by enzymatic digestion followed by treatment with Rho-associated kinase (ROCK) inhibitor Y-27632. The cell suspension of healthy iPS cells is nucleofected (Amaxa) with HB9:GFP BAC DNA, and the suspension of SMA iPS cells is nucleofected with HB9:mCherry BAC DNA. On day 4 following nucleofection, the cells from both groups undergo neomycin selection. Resulting iPS cell lines are then used for differentiation into motor neurons.

**Differentiation into MNs**: On Day 0 of differentiation, the human iPS cell lines (healthy and SMA) are plated in embryoid body (EB) media(KO DMEM (Invitrogen, catalog # 10829-018); 10% Knockout Serum Replacement (Invitrogen, catalog# A1099202); 10% Plasmanate (Talecris); 1% Glutamax (Invitrogen, catalog# 35050079); 1% non-essential amino acids (Invitrogen, cat#11140050)) in a six well plate at a density of 800,000 cells/well, for a total volume of 3 ml of EB medium per well. The cultures are supplemented with an additional 3 ml of EB medium three days later. On Days 5 and 8, embryoid bodies (EBs) are replated in each well of a 6 well plate in fresh EB medium. On Day 11, EBs are pre-induced to become motor neurons. EBs are resuspended in N2 Base (DMEM/F12 + Glutamax (Invitrogen, catalog# 10565); 1 % N-2 Supplement (Invitrogen, catalog# 17502-048), .16 % D-Glucose (Sigma, catalog# G8769); and 0.2 mM Ascorbic Acid (Sigma, catalog# A4403-100mG)), 1 uM Retinoic Acid (RA) (Sigma, cat. # R2625), and 100 nM Purmorphamine (Cayman Chemical, cat# 483367-10-8) and are replated in each well of a 6-well plate, for a total volume of 3 ml EB suspension per well. On Day 14, EBs are induced to become motor neurons by resuspending in N2 Base,1 uM RA, 1 uM Purmorphamine and plated at 5 ml of EB suspension per well. Cells are washed as needed until Day 28, at which point EBs are dissociated for motor neuron plating and maturation. EBs are resuspended in papain and triturate, and 2 ml of suspension is then plated in each well of a six-well plate. Dissociated EBs are then resuspended in Motor Neuron Maturation medium (DMEM/F12 + Glutamax (Invitrogen, catalog# 10565); 2% N-2 Supplement (Invitrogen, catalog# 17502-048); 4% B-27 Supplement (Invitrogen, catalog# 17504-044); .32% D-Glucose (Sigma, catalog# G8769); .4 mM Ascorbic Acid (Sigma, catalog# A4403-100mG); 2ng/mL GDNF (R&D, catalog# 212-GD); 2ng/mL BDNF (R&D, catalog# 248-BD); and 2ng/mL CNTF (R&D, catalog# 257-NT/CF), for a final concentration of 1.6 x 10⁶ cells per ml.

**Muscle Culture**:To make the healthy muscle culture, primary skeletal muscle progenitors (Cook Myosite) are expanded in growth medium (Cook Myosite). Cells are plated at high density (500,000-2,000,000 per well of a 6-well plate or equivalent) in differentiation medium (DMED/F12, 1% horse serum, 1% N2, 1 uM DAPT, 1-10 ug/ml laminin onto poly-1-lysine/laminin coated plates), and allowed to fuse and mature for up to 1 week. DAPT is removed and myotubes are grown in Neuron Maturation Medium. After 2-3 weeks, recombinant Agrin is added at 10 ng/ml to some cultures to induce acetylcholine clustering. Axons are monitored by confocal microscopy.

**Synaptic Competition**: An equal volume of cell suspension of Day-28-dissociated EB cells derived from healthy human subjects is combined with an equal volume of cell suspension of samples derived from SMA subjects and the combination is plated at a density of 5000 to 50,000 cells per well of a six-well plate, also containing healthy muscle culture, in Motor Neuron Maturation medium. The axons are monitored by confocal microscopy, which allows visualization of the retraction of axons (red) from SMA subjects as they are replaced by healthy axons (green) during synaptic competition.

### Example 2 (prophetic example) Co-cultures of motor neurons to model ALS

This example illustrates a co-culture of motor neurons (MN) derived from human iPS cells from healthy human subjects and human subjects having ALS ("ALS subjects") in order to model ALS disease. The iPS cells are derived from skin biopsies from 10 healthy subjects, 10 subjects with sporadic ALS, and subjects who carry mutations in SOD1 (e.g., SOD1G93A, SOD1G85R, or SOD1G37R), subjects who carry a mutation in TDP=43, and subjects who carry a SNP (e.g., rs12608932) in intron 21 of UNC13A at chromosome 19p13.3 or a SNP (e.g., rs2814707 or rs3849942) at chromosome 9p21.2, for a total of 10 subjects for each mutation. This example makes use of the competition assay described in Example 1.

The human iPS cells may either be normal or stably transfected with a fluorescent marker under the control of the promoter of a neuronal marker (e.g., HB9). In this example, healthy iPS cells are stably transfected with a vector capable of expressing GFP (green color) and the iPS cells from ALS subjects are stably transfected with a vector capable of expressing mCherry (red color) using the BAC method described in Example 1.

MNs are derived, combined with healthy muscle, and allowed to undergo synaptic competition, as described in Example 1. The axons are monitored by confocal microscopy, which allows visualization of the retraction of axons (red) from ALS subjects as they are replaced by healthy axons (green) during synaptic competition. Once the model is established, it can be used to screen for compounds that improve the activity of the MNs derived from the ALS subjects.

### Example 3 (prophetic example) Co-culture of neurons and astrocytes to model ALS

This example illustrates a co-culture of different combinations of motor neurons (MN) and astrocytes from human iPS cells. The iPS cells are derived from skin biopsies from 10 healthy subjects, 10 subjects with sporadic ALS, and subjects who carry mutations in SOD1 orTDP-43, mutations in gene (s) in linkage region at chromosome 9p21.2, or mutations in UNC13A at chromosome 19p13.3, for a total of 10 subjects for each mutation. Once the model is established, it can be used to identify the role played by MNs and astrocytes in causing MN cell death associated with ALS. The model can also be used to screen for compounds that rescue the phenotype by protecting or preventing cell death..

ALS and healthy iPS cell lines are genetically modified to each carry two reporters: ALDhL1-GFP to label astrocytes green and HB9-mCherry to label MN red. MN are derived from either the ALS or healthy iPS cell lines according to the methods described in Example 1. The MNs are also allowed to differentiate further into astrocytes. The MN and astrocytes are then sorted using fluorescent activated cell sorting (FACS) and replated in varying ratios (e.g., 5:1, 3:1, 1:1, 1:3, and 1:5). In order to model disease, the following combinations can be replated: (1) healthy MNs with healthy astrocytes; (2) healthy MNs with ALS astrocytes; (3) ALS MNs with healthy astrocytes; and (4) ALS MNs with ALS astrocytes. Axon degeneration and MN cell death is monitored by confocal imaging of red axons and cell bodies. The degeneration of red axons in close proximity to green astrocytes is also monitored.

### Example 4 (prophetic example) Co-culture of dopaminergic neurons and striatal GABAergic neurons to model PD

This example illustrates an *in vitro* model of PD that can be used to understand the basis of disease and for drug screening. iPSC lines are generated from skin biopsies obtained from 10 healthy control subjects with no known family history of PD, 10 patients with sporadic PD, patients each with mutations in the genes that encode α-synuclein (PARK1), parkin (PARK2), PINK 1 (PARK6), or LRRK2 (PARK8) for a total of 10 patients for each mutation ("mutant subjects"). iPS cells from each population are generated as described in Example 1, except that they are stably transfected with Pitx3:GFP, which is a reporter construct to identify dopaminergic fate, and with DARPP32:mcherry, which is a reporter construct to identify striatal fate. The iPS cells are then differentiated into dopaminergic neurons and striatal GAPAergic neurons. Dopaminergic neurons are obtained by differentiating the iPSCs according to the method of Chambers et al (2009) Nature Biotech 27(3) 275-280. Briefly, for neural induction, nearly-confluent plates of iPS cells are incubated with initial differentiation medium (knockout (K/O) serum replacement medium supplemented with 10 uM TGF-b inhibitor (Tocris) and 500 ng/ml of Noggin (R&D)). After five days of differentiation, the TGF-b is withdrawn and increasing amounts of N2 media (25%, 50%, 75%) is added to the K/O serum replacement medium every two days while maintaining 500 ng/ml of Noggin. Dopaminergic patterning is then initiated with the addition of sonic hedgehog on days 5-9, followed by the addition of BDNF, ascorbic acid, sonic hedgehog and FGF8. The dopaminergic neurons are then matured with BDNF, ascorbic acid, GDNF, TGFb3 and cAMP, and analyzed for expression of GFP in order to confirm activation of the Pitx3 promoter. As with the dopaminergic neurons, neural induction of the striatal neurons is achieved by the dual SMAD inhibition method described in Chambers et al (2009) Nature Biotech 27(3) 275-280. Cells are treated with SMAD inhibitors fro 11 days and then with shh agonist (SAG)(100-300nM) on day 7 for 2 weeks in order to ventralize them into striatal GABAergic neurons. Neurons are allowed to mature for 30-60 days. Striatal GABAergic neurons are analyzed for mCherry expression in order to confirm the activation of the DARPP32 promoter.

Next, the dopaminergic neurons are mixed with the GABAergic neurons at different ratios (1:4, 1:1, 4:1) and the co-culture is allowed to mature and form synapses in maturation medium. The dopaminergic neurons send axons (green) to, and form synapses with, striatal GABAergic neurons (red). Axons are monitored for degeneration by live imaging using an image express automated confocal microscope. Images are scored for the number of degenerating axons by measuring "blebbing" in the axons.

Once the model is established, compound libraries are screened and assayed for the ability to rescue the axon degeneration phenotype.

### Example 5 (prophetic example) Conditioned plates to model ALS

This example illustrates an *in vitro* model of ALS that can be used to understand the role played by extracellular matrix and other secreted proteins deposited by diseased neurons or astrocytes in the degeneration of MNs. The combinations of diseased/healthy motor neurons and astrocytes described in Example 3 are cultured on tissue culture plates for 2 to 20 days. The cells are scraped off manually with a cell scraper, such that extracellular matrix (ECM) remains on the plate. Each plate is then washed with PBS two times. Healthy motor neurons are then plated on the plates in fresh medium that is then routinely replaced. Cell death is monitored at different time points (0, 1, 2, 3, 4, 5, 10, 15, 20, 25, 30, 35, 40, 50 days) after initial plating by using any technique known in the art, for example by using activated caspase 3 antibody. Plates with ECM that appears to exert a neuroprotective effect or neurotoxic effect are identified, and the effect can then be traced back to the specific combination of cells, in order to determine which cell deposited the neuroprotective or neurotoxic ECM. Proteins of interest are then identified by routine biochemical analysis such as mass spectrometry.

### Example 6 (prophetic example) Co-culture of healthy pancreatic beta cells with diseased adipocytes or diseased skeletal muscle cells

This example illustrates an in vitro model of diabetes and obesity that can be used to understand the basis of disease and for drug screening. Human induced pluripotent stem (iPS) cell lines are derived from the following groups of human subjects: (1) healthy non-obese; (2) obese with Diabetes Type II (DTII); (3) obese without DTII; (4) non-obese with DTII; (5) obese with Diabetes Type I (DTI); (6) obese without DTI; and (7) non-obese with DTI using methods described in Example 1. Cells from each group are differentiated into adipocytes, pancreatic beta cells and skeletal muscle. To derive adipocytes, the methods provided in Taura et al. (2009) FEBS Letters 583: 1029-33 are followed. Briefly, EBs are formed from the iPS cells, in the presence of retinoic acid and / or BMP4 and Activin A. After 12 days, cells are transferred to Poly L-ornithine/fibronectin plates, followed by differentiation in the presence of DMEM-F12, 10% KSR or fetal calf serum or defined growth factors (BMP4, FGF, VEGF) and an adipogenic cocktail (IBMX, dexamethasone, insulin, indomethacin and pioglitazone). Alternatively, adipocytes are derived from each of the four groups of subjects following liposuction or other fat-removal procedure. To derive pancreatic beta cells, cells are first induced to become definitive endoderm by culturing in the presence of high concentrations of Activin A and low serum as described in D'Amour et al. (2005) Nature Biotechnol 23:1534-41, or high concentrations of Activin and low doses of Wnt3A. Differentiation is monitored using cell surface markers CXCR4 and CKIT and intracellular markers SOX17, FOXA2, FOXA3 and the absence of SOX7. The definitive endoderm is then specified to the pancreatic lineage using retinoic acid, Cyclopamine, Noggin, Exendin 4, DAPT and FGF10. Pancreatic differentiation is determined by monitoring PDX1 and NGN3 expression and insulin release after glucose stimulation. Skeletal muscle cells are derived as described in Barberi et al. (2007) Nature Medicine 13(5):642-648.

Following differentiation, cells are plated in low-serum (e.g., 2% Fetal Calf Serum) DMEM or RPMI with antibiotics in the following Assay combinations:
A. Group (1) pancreatic beta cells with Group (1) adipocytes
B. Group (1) pancreatic beta cells with Group (2) adipocytes
C. Group (1) pancreatic beta cells with Group (3) adipocytes
D. Group (1) pancreatic beta cells with Group (4) adipocytes
E. Group (1) pancreatic beta cells with Group (5) adipocytes
F. Group (1) pancreatic beta cells with Group (6) adipocytes
G. Group (1) pancreatic beta cells with Group (7) adipocytes
H. Group (1) pancreatic beta cells with Group (1) skeletal muscle cells
I. Group (1) pancreatic beta cells with Group (2) skeletal muscle cells
J. Group (1) pancreatic beta cells with Group (3) skeletal muscle cells
K. Group (1) pancreatic beta cells with Group (4) skeletal muscle cells
L. Group (1) pancreatic beta cells with Group (5) skeletal muscle cells
M. Group (1) pancreatic beta cells with Group (6) skeletal muscle cells
N. Group (1) pancreatic beta cells with Group (7) skeletal muscle cells

The co-cultures are then tested at various time points (0, 1, 2, 5, 10, 20, 30 days) for insulin-mediated glucose uptake by the adipocytes or the skeletal muscle cells. In this way, it can be determined which cell-type (e.g., pancreatic beta vs adipocytes or pancreatic beta vs skeletal muscle cells) is responsible for the disease and the effect on insulin-mediated glucose uptake. In addition, drug screening assays may be performed in order to screen for compounds that rescue impaired insulin-mediated glucose uptake.

### Example 7 (prophetic example) Co-cultures of diseased pancreatic beta cells with healthy adipocytes or healthy skeletal muscle cells

Human induced pluripotent stem (iPS) cell lines are derived from the following groups of human subjects: (1) healthy non-obese; (2) obese with Diabetes Type II (DTII); (3) obese without DTII; (4) non-obese with DTII; (5) obese with Diabetes Type I (DTI); (6) obese without DTI; and (7) non-obese with DTI using methods described in Example 1. Cells from each group are differentiated into adipocytes, pancreatic beta cells and skeletal muscle as described in Example 6. Alternatively, adipocytes are derived from each of the four groups of subjects following liposuction or other fat-removal procedure. Following differentiation, cells are plated in low-serum (e.g., 2% Fetal Calf Serum) DMEM or RPMI medium with antibiotics in the following Assay combinations:
A. Group (1) adipocytes with Group (1) pancreatic beta cells
B. Group (1) adipocytes with Group (2) pancreatic beta cells
C. Group (1) adipocytes with Group (3) pancreatic beta cells
D. Group (1) adipocytes with Group (4) pancreatic beta cells
E. Group (1) adipocytes with Group (5) pancreatic beta cells
F. Group (1) adipocytes with Group (6) pancreatic beta cells
G. Group (1) adipocytes with Group (7) pancreatic beta cells
H. Group (1) skeletal muscle cells with Group (1) pancreatic beta cells
I. Group (1) skeletal muscle cells with Group (2) pancreatic beta cells
J. Group (1) skeletal muscle cells with Group (3) pancreatic beta cells
K. Group (1) skeletal muscle cells with Group (4) pancreatic beta cells
L. Group (1) skeletal muscle cells with Group (5) pancreatic beta cells
M. Group (1) skeletal muscle cells with Group (6) pancreatic beta cells
N. Group (1) skeletal muscle cells with Group (7) pancreatic beta cells

The co-cultures are then tested at various time points (0, 1, 2, 5, 10, 20, 30 days) for insulin-mediated glucose uptake by the adipocytes or the skeletal muscle cells. In this way, it can be determined which cell-type (e.g., pancreatic beta vs adipocytes or pancreatic beta vs skeletal muscle cells) is responsible for the effect on insulin-mediated glucose uptake. In addition, drug screening assays may be performed in order to screen for compounds that rescue impaired insulin-mediated glucose uptake.

### Example 8 (prophetic example) Co-cultures of cortical neurons from healthy adults and sporadic Alzheimer's patients bearing a disease-associated SNP polymorphism

This experiment seeks to identify a presynaptic or postsynaptic defect associated with Alzheimer's disease in individuals carrying the rs3851179 allele, which has been associated with Alzheimer's disease in a genome wide association study (Harold et al (2009), Nature, 41(10):1088-1095). This allele is located within 8 kb of the 5' end of the phosphatidylinositol-binding clathrin assembly protein (PICALM) gene (GenBank Accession Nos: PCALM isoform 1 mRNA NM_007166.2; and PCALM isoform 2 mRNA NM_001008660.1). The PICALM protein has been shown to play a role in clathrin-mediated endocytosis (See, e.g., Yao et al (2005), J Comp Neurol, 481:58-69), a process that is critical to both presynaptic vesicle fusion and recycling, and postsynaptic receptor trafficking. Thus, it is of great interest to determine whether Alzheimer's patients bearing this polymorphism exhibit presynaptic, postsynaptic, or both presynaptic and postsynaptic functional or morphological deficits. Establishing, the synaptic locus of a neurodegenerative disorder could greatly facilitate the identification of relevant drug targets. This question can be readily studied by analyzing the morphology and function of the following types of synapses (synaptic combinations) that form in co-cultures of distinctly labeled cortical neurons differentiated from iPSCs derived from healthy and Alzhemer's patients:
(A) Healthy Presynaptic to Healthy Postsynaptic
(B) Alzheimer's Presynaptic to Alzheimer's Postsynaptic
(C) Healthy Presynaptic to Alzheimer's Postsynaptic
(D) Alzheimer's Presynaptic to Healthy Postsynaptic

A defect that is primarily presynaptic would be expected to be manifested in groups (B) and (D), but not (A) and (C). Conversely, a defect that is primarily postsynaptic would be manifested primarily in groups (B) and (C), but not (A) and (D).

Human induced pluripotent stem (iPS) cell lines are derived from the following groups of human subjects: (A) Twenty healthy individuals (age 65-85) genotyped for the absence of the rs3851179 SNP allele; (2) Twenty Alzheimer's patients (age 65 and above) genotyped for the presence of the rs 3851179 SNP allele.

Neural differentiation is carried out essentially as described in Gaspard et al (2008), Nature, 455(7211):351-357. In brief, iPSCs are trypsinized, dissociated and plated at a density of 5 x 10³ cells per cm² on gelatin-coated cell culture plastic dishes in ESC medium. After adhesion, medium is changed to defined neural differentiation medium (DNDM). DNDM consists of DMEM/F12 (Invitrogen-Gibco) supplemented with 1 N2 supplement (100 N2 supplement consists of 8.61 µM insulin, 1 mM transferrin, 2 µM progesterone, 10.01 mM putrescine and 3.01 µM selenite; Invitrogen-Gibco), 2 mM glutamine, 1X MEM-nonessential amino acids, 1 mM sodium pyruvate, 0.5 mg/ml bovine serum albumin (BSA) fraction V (all from Invitrogen-Gibco), and 110 µM -mercaptoethanol (Sigma). Cyclopamine (Calbiochem) is added from day 2 to day 10 in the differentiation medium at a final concentration of 1 µM. After 12 days of differentiation, cells derived from an Alzheimer's and Healthy subjects are transduced with lentiviruses encoding GFP and DS-Red (or other red fluorescednt protein). After 48 hours, GFP-expressing (Alzheimer's) and RFP-expressing cells are trypsinized, dissociated, mixed together at a 1:1 ratio and plated on polylysine/laminin (Becton-Dickinson) coated glass coverslips, and cultured for 30 days in N2B27 medium to allow synapse formation and maturation. N2B27 medium consists of a 1:1 mixture of DMEM/F 12 supplemented with 1 N2, 2 mM glutamine, 0.5 mg/ml BSA fraction V and 110 µM β-mercatoethanol with Neurobasal supplemented with B27 (without vitamin A; Invitrogen-Gibco) and 2 mM glutamine.

After 30 days in culture, cells are fixed and processed for immunofluorescence labeling for the presynaptic marker synaptophysin and for the GluR2 AMPA receptor subunit. Co-localized GluR2/synaptophysin puncta are then quantified by standard immunofluoresence imaging, by use of a filter set that can discriminate four separate wavelengths (i.e., separate channels for Healthy Neuron GFP, Alzheimer's neuron DS-Red, a synaptophysin-antibody flurophore, and a GluR2 AMPA subunit antibody fluorophore). The number of puncta for each synaptic combination (A-D as described above) is then quantified and compared for significant differences by Student's t-test.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention.

## Claims

1. A method of identifying a modulatory agent, the method comprising:
(a) co-culturing a first and a second population of differentiated cells in the presence or absence of a test agent, wherein the first population comprises differentiated cells derived from induced pluripotent stem cells (iPSCs) generated from cells of a first human subject identified as having, or predisposed to a neurodegenerative disorder, and wherein the second population comprises differentiated cells derived from a second human subject, wherein the differentiated cells in at least one of said populations comprise neurons, neural stem cells, or neural progenitors; and
(b) determining that the test agent is a modulatory agent if a cellular phenotype of the co-cultured differentiated cells of the first or second population is reduced or increased in the presence of the test agent.

2. The method of claim 1, wherein the second population does not comprise cells differentiated from human embryonic stem cells.

3. The method of claim 1, wherein in the co-cultured first and second populations the ratio of the differentiated cells of one cell type from the first population to differentiated cells of the one cell type from the second population is greater than 1:1, 10:1, 100:1, 1000:1, or 10,000:1.

4. The method of claim 1, wherein the neurodegenerative disorder is a sporadic form of a neurodegenerative disorder.

5. The method of claim 1, wherein at least one of the populations is detectably labeled.

6. The method of claim 1, wherein the at least one population comprises cells detectably labeled by expression of a fluorescent protein, staining with a fluorescent dye, staining with a fluorescently labeled antibody or staining with a fluorescently labeled protein with high affinity for a cell surface protein or receptor.

7. The method of claim 1, wherein one of the first and second populations is substantially enriched in a cell type relative to the other population.

8. The method of claim 1, wherein the cellular phenotype is one or more phenotypes selected from the group consisting of: survival, apoptosis, necrosis, axonal degeneration, axonal guidance, axonal morphology, dendritic morphology, receptor density, synaptogenesis, neurogenesis, synapse density, synaptic transmission, synaptic signaling, receptor trafficking, protein trafficking, protein aggregation, proteasome activity, receptor expression, oxidative stress (ROS), FGFR- signaling, FGF signaling, mitochondrial activity, mitochondrial distribution, mitochondrial morphology, and mRNA expression profile.

9. The method of claim 1, wherein the differentiated cells in the first population comprise neurons selected from the group consisting of: motor neurons, dopaminergic neurons, GABAergic neurons, cortical projection neurons, striatal neurons, and spinal cord motor neurons.

10. The method of claim 1, wherein the neurodegenerative disorder is selected from the group consisting of: Amyotrophic Lateral Sclerosis (ALS), Alzheimer's disease, Spinal Muscular Atrophy (SMA), Huntington's Disease, and Parkinson's Disease.

11. The method of claim 1, wherein the neurodegenerative disorder is selected from the group consisting of: Alexander's disease, Alper's disease, ataxia telangiectasia, Batten disease, bovine spongiform encephalopathy, Canavan disease, Cockayne syndrome, corticobasal degeneration, Creutzfeldt- Jakob disease, HIV-associated dementia, Kennedy's disease, Krabbe's disease, lewy body dementia, Machado-Joseph disease, multiple sclerosis, multiple system atrophy, narcolepsy, neuroborreliosis, Pelizaeus-Merzbacher Disease, Pick's disease, primary lateral sclerosis, prion diseases, Refsum's disease, Sandhoffs disease, Schilder's disease, subacute combined degeneration of spinal cord secondary to pernicious anaemia, schizophrenia, spinocerebellar ataxia, Steele-Richardson-Olszewski disease, and tabes dorsalis.

12. A co-culture composition comprising a first and a second population of differentiated cells, wherein the first population comprises differentiated cells derived from induced pluripotent stem cells (iPSCs) derived from a first human subject, wherein the second population comprises differentiated cells derived from a second human subject, wherein the differentiated cells in at least one of said populations comprise neurons, neural stem cells, or neural progenitors, and wherein the first or second human subject is identified as having, or predisposed to a neurodegenerative disorder.

13. The co-culture composition of claim 12, wherein the second population does not comprise cells differentiated from human embryonic stem cells.

14. The co-culture composition of claim 12, wherein at least one of the populations is detectably labeled.

15. The method of claim 1, or the co-culture of claim 13, wherein the neurodegenerative disorder does not comprise amyotrophic lateral sclerosis caused by a SOD^{G37R} mutation, a SOD1^{G93A} mutation, or a SOD1^{G85R} mutation.

## Patentansprüche

1. Ein Verfahren zum Identifizieren eines modulatorischen Agens, wobei das Verfahren umfasst:
(a) das gemeinsame Kultivieren einer ersten und einer zweiten Population von differenzierten Zellen in der Gegenwart oder Abwesenheit eines Testagens, wobei die erste Population differenzierte Zellen umfasst, die von induzierten pluripotenten Stammzellen (iPSCs) abgeleitet sind, die aus Zellen von einem ersten menschlichen Patienten erzeugt wurden, bei dem eine neurodegenerative Störung identifiziert wurde oder der eine Prädisposition für eine solche aufweist, und wobei die zweite Population differenzierte Zellen umfasst, die von einem zweiten menschlichen Patienten abgeleitet sind, wobei die differenzierten Zellen bei wenigstens einer der Populationen Neuronen, neurale Stammzellen oder neurale Progenitoren umfassen; und
(b) das Feststellen, dass das Testagens ein modulatorisches Agens ist, wenn ein zellulärer Phänotyp der gemeinsam kultivierten differenzierten Zellen der ersten oder zweiten Population in der Gegenwart des Testagens vermindert oder verstärkt wird.

2. Das Verfahren nach Anspruch 1, wobei die zweite Population keine Zellen umfasst, die sich aus menschlichen embryonalen Stammzellen differenziert haben.

3. Das Verfahren nach Anspruch 1, wobei bei den gemeinsam kultivierten ersten und zweiten Populationen das Verhältnis der differenzierten Zellen von einem Zelltyp aus der ersten Population zu differenzierten Zellen des einen Zelltyps aus der zweiten Population größer als 1:1, 10:1, 100:1, 1000:1 oder 10.000:1 ist.

4. Das Verfahren nach Anspruch 1, wobei die neurodegenerative Störung eine sporadische Form einer neurodegenerativen Störung ist.

5. Das Verfahren nach Anspruch 1, wobei wenigstens eine der Populationen nachweisbar markiert ist.

6. Das Verfahren nach Anspruch 1, wobei die wenigstens eine Population Zellen umfasst, die nachweisbar markiert sind durch Expression eines fluoreszierenden Proteins, Anfärben mit einem fluoreszierenden Farbstoff, Anfärben mit einem fluoreszenzmarkierten Antikörper oder Anfärben mit einem fluoreszenzmarkierten Protein mit hoher Affinität für ein Zelloberflächenprotein oder einen Rezeptor.

7. Das Verfahren nach Anspruch 1, wobei eine der ersten und zweiten Populationen im Hinblick auf einen Zelltyp in Bezug auf die andere Population wesentlich angereichert ist.

8. Das Verfahren nach Anspruch 1, wobei der zelluläre Phänotyp einer oder mehrere der Phänotypen ist, die ausgewählt sind aus der Gruppe, bestehend aus: Überleben, Apoptose, Nekrose, axonaler Degeneration, axonaler Leitung, axonaler Morphologie, dendritischer Morphologie, Rezeptordichte, Synaptogenese, Neurogenese, Synapsendichte, synaptischer Transmission, synaptischer Signalgebung, Rezeptor-Trafficking, Protein-Trafficking, Proteinaggregation, Proteasomenaktivität, Rezeptorexpression, oxidativem Stress (ROS), FGFR-Signalgebung, FGF-Signalgebung, mitochondrialer Aktivität, mitochondrialer Verteilung, mitochondrialer Morphologie und mRNA-Expressionsprofil.

9. Das Verfahren nach Anspruch 1, wobei die differenzierten Zellen in der ersten Population Neuronen umfassen, die ausgewählt sind aus der Gruppe, bestehend aus: Motoneuronen, dopaminergen Neuronen, GABAergen Neuronen, kortikalen Projektionsneuronen, striatalen Neuronen und Rückenmarks-Motoneuronen.

10. Das Verfahren nach Anspruch 1, wobei die neurodegenerative Störung ausgewählt ist aus der Gruppe, bestehend aus: Amyotropher Lateralsklerose (ALS), Alzheimer-Krankheit, spinalmuskulärer Atrophie (SMA), Huntington's Krankheit und Parkinson-Krankheit.

11. Das Verfahren nach Anspruch 1, wobei die neurodegenerative Störung ausgewählt ist aus der Gruppe, bestehend aus: Alexanders Krankheit, Alpers Krankheit, Ataxia telangiectasia, Batten-Krankheit, boviner spongiformer Enzephalopathie, Canavan-Krankheit, Cockayne-Syndrom, kortikobasaler Degeneration, Creutzfeldt-Jakob-Krankheit, mit HIV verbundener Demenz, Kennedy-Krankheit, Krabbe-Krankheit, Lewy-Körper-Demenz, MachadoJoseph-Krankheit, multipler Sklerose, multipler Systematrophie, Narkolepsie, Neuroborreliose, Pelizaeus-Merzbacher-Krankheit, Pick's Krankheit, primärer Lateralsklerose, Prionkrankheiten, Refsum's Krankheit, Sandhoffs Krankheit, Schilder's Krankheit, subakuter kombinierter Degeneration des Rückenmarks begleitend zu perniziöser Anämie, Schizophrenie, spinozerebellarer Ataxie, Steele-Richardson-Olszewski-Krankheit und Tabes dorsalis.

12. Eine Zusammensetzung zur gemeinsamen Kultivierung, welche eine erste und eine zweite Population von differenzierten Zellen umfasst, wobei die erste Population differenzierte Zellen umfasst, die von induzierten pluripotenten Stammzellen (iPSCs) abgeleitet sind, die von einem ersten menschlichen Patienten abgeleitet sind, wobei die zweite Population differenzierte Zellen umfasst, die von einem zweiten menschlichen Patienten abgeleitet sind, wobei die differenzierten Zellen bei wenigstens einer der Populationen Neuronen, neurale Stammzellen oder neurale Progenitoren umfassen und wobei bei dem ersten oder zweiten menschlichen Patienten eine neurodegenerative Störung identifiziert wurde oder dieser eine Prädisposition für eine solche aufweist.

13. Die Zusammensetzung zur gemeinsamen Kultivierung nach Anspruch 12, wobei die zweite Population keine Zellen umfasst, die sich aus menschlichen embryonalen Stammzellen differenziert haben.

14. Die Zusammensetzung zur gemeinsamen Kultivierung nach Anspruch 12, wobei wenigstens eine der Populationen nachweisbar markiert ist.

15. Das Verfahren nach Anspruch 1 oder die gemeinsame Kultivierung nach Anspruch 13, wobei die neurodegenerative Störung keine amyotrophe Lateralsklerose umfasst, die durch eine SOD^{G37R}-Mutation, eine SOD1^{G93A}-Mutation oder eine SOD1^{G85R}-Mutation verursacht wird.

## Revendications

1. Procédé d'identification d'un agent modulateur, le procédé consistant à :
(a) co-cultiver une première et une seconde population de cellules différenciées par la présence ou par l'absence d'un agent de test, où la première population comprend des cellules différenciées dérivées de cellules souches pluripotentes induites (CSPi) générées à partir de cellules d'un premier sujet humain identifié comme présentant ou étant prédisposé à un trouble neurodégénératif, et où la seconde population comprend des cellules différenciées dérivées d'un second sujet humain, où les cellules différenciées dans l'au moins une desdites populations comprennent des neurones, des cellules souches neurales, ou des progéniteurs neuraux ; et
(b) déterminer que l'agent de test est un agent modulateur si un phénotype cellulaire des cellules différenciées co-cultivées de la première ou de la seconde population est réduit ou accru en présence de l'agent de test.

2. Procédé selon la revendication 1, dans lequel la seconde population ne comprend pas de cellules différenciées provenant de cellules souches embryonnaires humaines.

3. Procédé selon la revendication 1, où dans la première et la seconde population co-cultivées, le ratio des cellules différenciées d'un type cellulaire de la première population pour les cellules différenciées du type cellulaire de la seconde population est supérieur à 1:1, 10:1, 100:1, 1 000:1 ou 10 000:1.

4. Procédé selon la revendication 1, dans lequel le trouble neurodégénératif est une forme sporadique d'un trouble neurodégénératif.

5. Procédé selon la revendication 1, dans lequel au moins une des populations est marquée de manière détectable.

6. Procédé selon la revendication 1, dans lequel la au moins une population comprend des cellules marquées de manière détectable par l'expression d'une protéine fluorescente, une coloration avec un colorant fluorescent, une coloration avec un anticorps marqué par fluorescence ou une coloration avec une protéine marquée par fluorescence ayant une haute affinité pour une protéine ou un récepteur de surface cellulaire.

7. Procédé selon la revendication 1, dans lequel une de la première et de la seconde population est essentiellement enrichie en un type cellulaire par rapport à l'autre population.

8. Procédé selon la revendication 1, dans lequel le phénotype cellulaire est un ou plusieurs phénotypes choisis parmi le groupe comprenant : la survie, l'apoptose, la nécrose, la dégénérescence axonale, le guidage axonal, la morphologie axonale, la morphologie dendritique, la densité en récepteurs, la synaptogenèse, la neurogenèse, la densité synaptique, la transmission synaptique, la signalisation synaptique, le trafic de récepteurs, le trafic de protéines, l'agrégation de protéines, l'activité du protéasome, l'expression de récepteurs, le stress oxydant (ROS), la signalisation du FGFR, la signalisation du FGF, l'activité mitochondriale, la distribution mitochondriale, la morphologie mitochondriale et le profil d'expression de l'ARNm.

9. Procédé selon la revendication 1, dans lequel les cellules différenciées dans la première population comprennent des neurones choisis parmi le groupe comprenant : des neurones moteurs, des neurones dopaminergiques, des neurones GABAergiques, des neurones de projection corticaux, des neurones striataux et des neurones moteurs de la moelle épinière.

10. Procédé selon la revendication 1, dans lequel le trouble neurodégénératif est choisi parmi le groupe comprenant : la sclérose latérale amyotrophique (SLA), la maladie d'Alzheimer, l'amyotrophie spinale (AMS), la maladie de Huntington et la maladie de Parkinson.

11. Procédé selon la revendication 1, dans lequel le trouble neurodégénératif est choisi parmi le groupe comprenant : la maladie d'Alexander, la maladie d'Alpers, l'ataxie-télangiectasie, la maladie de Batten, l'encéphalopathie spongiforme bovine, la maladie de Canavan, le syndrome de Cockayne, la dégénérescence cortico-basale, la maladie de Creutzfeldt-Jakob, la démence associée au VIH, la maladie de Kennedy, la maladie de Krabbe, la démence à corps de Lewy, la maladie de Machado-Joseph, la sclérose en plaques, l'atrophie multisystématisée, la narcolepsie, la neuroborréliose, la maladie de Pelizaeus-Merzbacher, la maladie de Pick, la sclérose latérale primitive, des maladies à prion, la maladie de Refsum, la maladie de Sandhoff, la maladie de Schilder, une dégénérescence combinée subaiguë de la moelle épinière secondaire à une anémie pernicieuse, la schizophrénie, l'ataxie spinocérébelleuse, la maladie de Steele-Richardson-Olszewski et l'ataxie locomotrice progressive.

12. Composition de co-culture comprenant une première et une seconde population de cellules différenciées, où la première population comprend des cellules différenciées dérivées provenant de cellules souches pluripotentes induites (CSPi) dérivées provenant d'un premier sujet humain, où la seconde population comprend des cellules différenciées dérivées d'un second sujet humain, où les cellules différenciées dans au moins l'une desdites populations comprennent des neurones, des cellules souches neurales ou des progéniteurs neuraux, et où le premier ou le second sujet humain est identifié comme présentant ou étant prédisposé à un trouble neurodégénératif.

13. Composition de co-culture selon la revendication 12, dans laquelle la seconde population ne comprend pas de cellules différenciées provenant de cellules souches embryonnaires humaines.

14. Composition de co-culture selon la revendication 12, dans laquelle au moins une des populations est marquée de manière détectable.

15. Procédé selon la revendication 1, ou co-culture selon la revendication 13, où le trouble neurodégénératif ne comprend pas de sclérose latérale amyotrophique provoquée par une mutation SOD^{G37R}, une mutation SOD1^{G93A} ou une mutation SOD1^{G85R}.
